(19) Europäisches Patentamt
European Patent Office
Office européen des brevets

(11) Publication number: **0 229 712 A2**

(12) # EUROPEAN PATENT APPLICATION

(21) Application number: 87300274.5

(22) Date of filing: 13.01.87

(51) Int. Cl.⁴: C 12 N 15/00
C 12 N 1/20, C 12 P 21/02
//(C12N1/20,C12R1:07)

(30) Priority: 13.01.86 US 824642

(43) Date of publication of application:
22.07.87 Bulletin 87/30

(84) Designated Contracting States:
AT BE CH DE FR GB IT LI LU NL SE

(71) Applicant: GENEX CORPORATION
16020, Industrial Drive
Gaithersburg Maryland 20877 (US)

(72) Inventor: Saunders, Charles Winston
13614 Duhart Road
Germantown Maryland 20877 (US)

Guyer, Mark Samuel
5710 Roosevelt Street
Bethesda Maryland 20817 (US)

(74) Representative: Holmes, Michael John
Frank B. Dehn & Co. European Patent Attorneys Imperial House 15-19 Kingsway
London, WC2B 6UZ, (GB)

(54) **The production of human serum albumin in bacillus.**

(57) A protein which reacts with an antibody to human serum albumin (HSA) can be synthesized in a Bacillus host and secreted therefrom. A Bacillus host is transformed with an expression vector comprising a structural gene which encodes HSA fused in correct reading frame, either directly or via a linker sequence, to the expression elements and signal sequence coding region of a gene that can be recognized by the transcription and translation systems of the host, such that said expression elements control the expression of the HSA-encoding sequence. The transformed strain is grown in a culture medium and subjected to conditions under which the gene fusion is expressed. The resultant protein can be secreted from the Bacillus host into the culture medium.

EP 0 229 712 A2

**Description**

# THE PRODUCTION OF HUMAN SERUM ALBUMIN IN BACILLUS

This invention relates to methods for synthesizing in microorganisms of the genus Bacillus and secreting therefrom a protein comprising the amino acid sequence encoded by a heterolgous gene. More specifically, this invention relates to such methods for synthesizing and secreting human serum albumin.

Recombinant DNA technology has been applied to the construction of microbial strains, and other cellular systems, which can make high levels of one or more proteins of interest which otherwise would have to be isolated from natural sources. These proteins may be valuable for either commercial or research purposes and may be of homologous origin (encoded by a gene from the host organism) or of heterologous origin (encoded by a gene from an organism other than the host).

To achieve the synthesis of such a genetically engineered protein, a microorganism, such as a bacterium, is first transformed with DNA containing a gene which encodes the protein of interest. This gene is operably linked to a regulatory sequence of DNA, including a transcription promoter, which is capable of directing expression of the gene in that bacterial host. In many instances, a heterologous gene is not efficiently expressed in such cases in a particular host because its natural promoter and/or translation initiation site cannot be efficiently recognized by the gene expression systems of the heterologous host. For the heterolgous gene to be efficiently expressed, its natural expression signals must be replaced, using recombinant DNA technology, by signals which can be recognized by the new host. Gene fusions also can be used to alter the properties of gene products of interest in other ways, such as to affect or optimize the ability of a protein to be secreted or to alter the biochemical or biophysical properties of a protein to make purification easier.

It is generally thought that proteins which are secreted from their native host may not fold properly if synthesized and accumulated intracellularly. Secretion of such proteins may be required to allow the formation of the disulfide bonds which are found in many secreted proteins. Disulfide bonds are generally thought not to form in the reducing environment within the cell. To eliminate the in vitro protein folding that is required for generation of activity of many such heterologous proteins accumulated intracellularly as inactive protein, many research groups are trying to effect secretion of such proteins from recombinant hosts.

Until fairly recently, the vast majority of genetic engineering experiments have employed Escherichia coli as the host microorganism. This is due to the fact that E. coli is one of the most genetically well characterized microorganisms and methods for stable transformation and growth have been developed. However, there are certain drawbacks associated with the use of E. coli as a host microorganism. Prominent among these is the fact that E. coli is generally incapable of secreting the protein product into the culture medium, and secreted proteins accumulate in the periplasm and membranes. Consequently, recovery of the product requires that the cells be recovered and the product separated from cellular matter, which can be a time-consuming and laborious process. Moreover, E. coli produces endotoxins which can contaminate the desired heterologous protein. Consequently, scrupulous care must be exercised in the purification of the product to ensure that it is free of endotoxins, particularly where the end product is to be employed in foods or medicines.

Because of the problems associated with the use of E. coli as a host, interest has developed in using other microbial hosts such as those of the genus Bacillus. In particular, Bacillus subtilis has been a focus of interest because it is a non-pathogenic organism which does not produce endotoxins and because it is capable of secreting protein into the surrounding medium.

One protein of interest is human serum albumin (HSA). HSA is the major protein component of plasma. HSA is secreted from the liver and contains 17 disulfide bonds (Behrens, et al., Fed. Proc. 34:591 [1975]). HSA is responsible for maintaining normal osmolarity in the bloodstream and acts as a carrier for various small molecules. HSA conventionally has been purified from blood for use in research and clinical studies. There are a number of reasons why it would be advantageous to produce HSA from a microorganism. Production of HSA from a microorganism would reduce the likelihood of transmitting infectious diseases, such as AIDS, upon treatment with HSA; it may be a more economical means of producing the protein; and it would minimize the reliance of blood supplies as the source of protein.

Throughout this application, the term HSA will be used to refer to serum albumin isolated from humans or a protein having the same amino acid sequence isolated from a microorganism. Reference also will be made to HSA cross-reacting material (CRM). This term will refer to a protein produced by a microorganism, with an amino acid sequence that has not yet been verified, which reacts with an HSA antibody.

HSA CRM has been synthesized and accumulated intracellularly by strains of E. coli which were developed using recombinant DNA technology. The synthesis of HSA at modest levels has been reported in the literature. In one case, the E. coli trp (tryptophan operon) regulatory region was used to effect transcription of the HSA gene (Lawn, R.M. et al., Nucl. Acids Res. 9:6103 [1981]). In view of the aforementioned problems associated with intracellular accumulation of normally secreted proteins and the potential advantages of effecting secretion, particularly by using a Bacillus strain as a host, it would be highly desirable to develop a system for synthesizing HSA in, and secreting it from, a Bacillus such as B. subtilis.

Accordingly, it is an object of the present invention to construct expression systems such that HSA will be synthesized in, and secreted from, B. subtilis.

Additional objects of this invention will become apparent to those skilled in the art upon reading the description of the invention below, taken in conjunction with the accompanying claims and figures.

In accordance with the present invention, there are disclosed methods for synthesizing in a Bacillus strain and secreting therefrom a protein that reacts with an antibody to HSA. A Bacillus host is transformed with a vector containing a gene which encodes human serum albumin fused to the promoter and, in-frame, to the signal sequence coding region of a gene the expression signals of which can be recognized by the host. The transformed strain is grown and subjected to conditions under which the gene fusion is expressed. Methods also are disclosed by which the resultant gene product is secreted into the growth medium and recovered therefrom.

## Brief Description of the Figures

Figures l, 2 and 3 are schematic representations of the construction of vectors for the manipulation of a nucleotide sequence encoding human serum albumin.

Figures 4 and 5 are schematic representations of the construction of two vectors each comprising a segment of the α-amylase gene of B. amyloliquefaciens (amy[BamP]). In pGX2472 the amy[BamP] segment comprises the promoter and coding region for the entire α-amylase signal sequence. In pGX2473 the amy[BamP] segment additionally specifies the three amino-terminal amino acids of extracellular α-amylase.

Figure 6 further illustrates the structures of the amy[BamP] segments in pGX2472 and pGX2473.

Figure 7 is a schematic representation of the construction of vector pGX2464 which comprises the amy[BamP]/hsa gene fusion designated ahs-l.

Figure 8 is a schematic representation of the construction of a vector and transformation of a strain therewith such that the transformed host carries an amy[BamP]/hsa gene fusion on its chromosome.

Figure 9 is a schematic representation of the construction of two vectors, pGX3804 and pGX3805, which contain the origin of replication of the filamentous coliphage f1. Figure 9 also illustrates the construction of a vector comprising the ahs-l gene fusion, the promoter distal noncoding sequence of which has been modified by oligonucleotide directed mutagenesis such that a transcriptional terminator is placed promoter-distal to the hsa-translation stop codon coding sequence.

Figure l0 illustrates the oligonucleotide mutagenesis used to create an amy[BamP]/hsa gene fusion in which the α-amylase signal sequence coding region is precisely joined to the DNA specifying proHSA.

Figure ll is a diagram of the structures of four amy/hsa fusions. In each structure amy[BamP] comprises the stippled area, hsa the white area, and synthetic linker sequences the black area. The sequence of the fusion area is indicated. For the DNA sequence, upper case letters represent amy[BamP]; upper case boldface letters represent mature HSA coding sequence; lower case italicized letters represent synthetic linker sequences; and lower case letters represent pro (of proHSA) coding sequences. For the amino acid sequence, lower case letters represent amylase sequence; lower case bold letters represent HSA sequence; and lower case italicized letters represent linker encoded amino acids.

Figure l2 is a schematic representation of the construction of a vector comprising an npr[BamP]/hsa gene fusion.

Figure l3 is a photograph showing protein bands resulting from the electrophoresis of cell and supernatant samples on a polyacrylamide gel. The samples were obtained from a B. subtilis strain transformed with a vector comprising ahs-l which was cultured under HSA producing conditions in 2% yeast extract. Lane A represents the cell sample; Lane B represents the supernatant sample which was trichloracetic acid (TCA) concentrated; Lane C, the supernatant sample which was Centricon concentrated; and Lane D, commercially obtained HSA mixed with supernatant from a negative control strain.

Figure l4 is a photograph showing protein bands resulting from the electrophoresis of pulse-labeled cell samples on a polyacrylamide gel. Proteins of about 68,000 Kd were isolated from three strains, but not from a negative control strain.

Figure l5 is a photograph showing protein bands resulting from polyacrylamide gel electrophoresis of pulse-labeled immunoprecipitated samples of three derivatives of strain BRl5l.

Figure l6 is a photograph showing protein bands resulting from the electrophoresis of immunoprecipitated cell samples on a polyacrylamide gel. The photograph shows the results of phenylethyl alcohol treatment of the strains.

Figure l7 is a photograph showing protein bands resulting from the electrophoresis of immunoprecipitated pulse-labeled protoplasts.

Figure l8 is a photograph showing protein bands resulting from the Western blot analysis of cell and supernatant samples obtained from two B. subtilis strains transformed with vectors carrying the ahs-l gene fusion One strain was chemically mutagenized.

The present invention relates to methods of synthesizing in cells of a Bacillus species, and secreting therefrom, a protein which reacts with an antibody to HSA. The method involves constructing an expression vector which comprises the gene encoding HSA which is fused to the expression elements and, in the correct reading frame, to the signal sequence coding region of a gene that can be recognized by the Bacillus host and transforming the host with the recombinant expression vector. The transformed host cells are grown under HSA-producing conditions.

The expression vectors of the invention can be constructed by inserting a hybrid gene into a vector which is capable of transformation and stable maintenance in the Bacillus host. The hybrid gene encodes HSA fused in-frame to the signal sequence coding region and to the regulatory region of a gene that can be recognized by the transcription and translation systems of the Bacillus host. When the host is Bacillus subtilis, the signal sequence coding region and regulatory region may be those of an α-amylase gene from B. amyloliquefaciens. (the gene referred to hereinafter as amy[BamP]). or of an extracellular neutral protease gene from B. amyloliquefaciens (the gene, referred to hereinafter as npr[BamP]).

To construct the amy[BamP]/hsa gene fusions (referred to hereinafter as ahs fusions). amy[BamP] is modified by conventional techniques (Maniatis et al., Molecular Cloning. A Laboratory Manual. Cold Spring Harbor Laboratory, Cold Spring Harbor. NY [1982]) such that it can be fused to a DNA sequence encoding HSA. amy[BamP] is modified by placing a restriction enzyme recognition sequence promoter-distal to the signal sequence coding region such that the expression elements (promoter. ribosome binding site and translation initiation codon) and coding sequence for the secretion signal sequence are contained within a restriction fragment that can be readily sub-cloned. This restriction site then can be used to fuse a heterologous gene to the amy[BamP] expression signals and signal sequence coding region. Two examples of vectors containing such modified amy[BamP] genes are shown in Figure 5. The vector designated pGX2472 contains a segment of amy[BamP] including the promoter and coding region for the entire signal sequence. The vector designated pGX2473 additionally contains the amy-[BamP] sequence that specifies the three amino-terminal amino acids of extracellular α-amylase. The construction of these and other vectors is described in detail in the examples below.

The gene coding for HSA is contained in pGX1042, which is harbored by E. coli K12 GX1170. The transformed strain has been deposited with Northern Regional Research Laboratories. Peoria. Illinois as NRRL No. B-15783. The amino terminus coding region of the HSA gene is modified to contain a restriction enzyme recognition site identical to the one created in the amy[BamP] sequence above and located so as to put the amy and hsa sequences into the same reading frame. A preferred source of the HSA gene is a vector designated pGX2451, which contains the DNA sequence for wild-type prepro-HSA modified by oligonucleotide directed mutagenesis such that an XhoI restriction endonuclease cleavage site overlaps the coding sequence for the amino terminus of mature HSA. This gene, designated hsa-3, then can be further modified such that it can be fused to the amy[BamP] sequence. To enable fusion with the amy[BamP] sequences described above, for example, the hsa-3 sequence can be modified by cloning a synthetic oligonucleotide containing a HindIII site into the XhoI site. A vector containing this DNA sequence, referred to as hsa-4, has been designated pGX2461.

The modified α-amylase gene and modified HSA gene may be fused together in accordance with conventional techniques. For example, to join hsa-4 of pGX2461 with the amy[BamP] segment present in pGX2447 (a derivative of pGX2472), as illustrated in Figure 7, the vectors can be digested with ClaI and HindIII restriction enzymes, the resulting fragments can be ligated, and the ligation mix can be used to transform B. subtilis BR151 cells. A resultant vector is designated pGX2464. The amy/hsa gene fusion in this vector is designated ahs-1, and the transformed strain is designated GX7003.

The ahs-1 gene fusion can be further modified. It specifies four amino acids between the α-amylase and HSA components of the fusion protein, three of which are encoded by the HindIII linker and the fourth of which is part of the HSA propeptide. Oligonucleotide-directed mutagenesis can be used to create a fusion in which the α-amylase signal sequence coding region is precisely joined to the DNA specifying the first amino acid of mature HSA. This gene fusion, designated ahs-3, is shown in Figure 11. The ahs-1 fusion also can be modified such that it will specify the α-amylase signal sequence and proHSA, the precursor form of HSA which is believed to be secreted through the membrane of the endoplasmic reticulum of liver cells. This gene fusion, also illustrated in Figure 11, is designated ahs-4. The construction of these gene fusions is described in detail in the examples section below.

As an alternative to constructing ahs fusions, gene fusions can be constructed wherein the promoter and signal sequence coding region are provided by a neutral protease gene (npr[BamP]) from B. amyloliquefaciens (Vasantha et al., J. Bacteriol. 159:811-819 [1984]). To construct the npr[BamP]/hsa gene fusion, npr[BamP] also is modified such that it can be joined to a DNA sequence encoding HSA. Using procedures similar to those described above for modifying amy[BamP], npr[BamP] is modified by inserting a restriction enzyme recognition site between the DNA sequences specifying the second and third amino acids on the carboxy side of the putative neutral protease signal sequence. The npr[BamP] expression signals and signal sequence coding region then can be fused to a modified hsa gene as described above. A detailed description of vector constructions is provided in the examples section of this application, below.

B. subtilis strains are transformed with vectors carrying the amy/hsa and npr/hsa gene fusions described above. Transformation can be effected using known techniques (Contente, S. and Dubnau, D., Plasmid, 2:555-571 [1979] and Chang and Cohen, Mol. Gen. Genet. 168: 111 [1979]). Depending upon the nature and characteristics of the vector carrying the gene fusion, the transformed host strain may carry the gene fusion either on a plasmid or in its chromosome. Detailed descriptions of the generation of both chromosomally-borne gene fusions and plasmid-borne gene fusions are set forth in the examples section below.

The transformants are grown under conditions conducive to synthesis of the protein product of the gene fusions. The transformed strains synthesize at substantial levels a protein having HSA antigenicity. In contrast, a strain harboring a plasmid containing hsa but lacking the amy or npr gene expression and signal sequence coding regions has not been found to synthesize such a protein.

Applicants have found that, under certain conditions, when strains of B. subtilis transformed with vectors carrying ahs fusions have been grown in 2% yeast extract, substantial amounts of HSA cross reacting material (CRM) can be detected in the culture supernatant.

Analysis of the total protein accumulated indicated that the extracellular location of the HSA CRM was not the result of release of intracellular material by cell lysis. This conclusion was drawn from the demonstration that the extracellular HSA CRM did not contain the signal sequence. Proteins to be exported typically are synthesized in the form of a precursor which contains an additional N-terminal peptide, termed a signal sequence, of typically 20-40 amino acids. The signal sequence usually is removed from the precursor during secretion of the protein. In addition, physiological or genetic modifications that prevent signal sequence removal prevent secretion. Thus, signal sequence removal is an indication that a protein has been secreted across a cell membrane. The amy/hsa and npr/hsa fusions specify protein products having a signal sequence from α-amylase and neutral protease, respectively, which function in B. subtilis. The procedure for determining whether the signal sequence has been removed from the protein is set forth in detail in the examples section below.

In contrast to the results obtained with cultures grown in 2% yeast extract, cells growing in minimal medium behave differently. When such HSA CRM-producing cells are pulse-labeled with a radioactive amino acid and centrifuged, the labeled HSA CRM is found primarily in the cell pellet. Typically, less than about 5% of the HSA CRM can be recovered from the supernatant of these cultures. However, when the HSA-producing cells are first converted to protoplasts by lysozyme treatment and then pulse-labeled, much of the HSA CRM is found in the culture supernatant, and the supernatant is enriched for the HSA CRM. These results demonstrate that HSA CRM can be secreted from B. subtilis protoplasts. As HSA CRM can be secreted from pulse-labeled cells only when the cell wall has been removed, it appears that HSA is prevented from reaching the culture medium by a component of the cell wall, at least under these conditions of growth and pulse-labeling.

From the pulse-labeling experiments, it appears that there is an inverse correlation between the fraction of HSA CRM from which the signal sequence has been removed and the relative amounts of HSA CRM produced by a transformed B. subtilis strain. This has been observed in pulse-labeling experiments with strains containing ahs-l or npr/hsa. For example, when the npr/hsa gene fusion is carried on a plasmid, the level of gene expression is relatively high and the HSA CRM has a gel electrophoretic mobility indicating that the signal sequence has not been removed. When the npr/hsa gene fusion is borne in the host chromosome, however, the level of expression is lower, but the majority of the HSA CRM produced is secreted from protoplasts and has had the signal sequence removed, as judged by mobility in gel electrophoresis experiments.

It is desirable to increase HSA production level. To increase the amount of HSA CRM produced, a transcription terminator can be inserted promoter distal to the amy/hsa or npr/hsa fusions, within 50 bp of the translational termination codon coding sequence. The presence of a transcription terminator is known to have a positive effect on the level of expression of some cloned genes, presumably by stabilizing the mRNA (Chernajousky et al., Ann. N.Y. Acad. Sci. 413:88 [1983]). Both the B. amyloliquefaciens neutral protease and α-amylase genes contain, at their 3′ end, a 22-42 base pair palindromic sequence which culminates in a string of thymidylate residues in the non-coding strand. These sequences resemble known transcriptional terminators from Escherichia coli and generally begin within 7 base pairs of the translation termination codon coding sequence.

A transcription termination sequence can be provided at the promoter distal end of amy/hsa or npr/hsa by first synthesizing an oligonucleotide that is analogous to these known sequences by providing a long inverted repeat sequence followed by a string of thymidylate residues. This synthetic "terminator" sequence is inserted promoter-distal to one of the gene fusions described above such that it begins within 10 base pairs of the translational stop codon coding sequence of hsa. A Bacillus strain transformed with a vector carrying this modified gene fusion and grown under HSA-producing conditions synthesizes enhanced levels of HSA in comparison to strains transformed with vectors carrying gene fusions which have not been so modified.

The level of accumulated HSA also can be increased by using a chemically mutagenized strain of Bacillus as host. It has been found that nitrosoguanidine treatment of a Bacillus strain transformed with an expression vector of this invention produces mutants having increased levels of accumulated HSA in comparison to the same strain before treatment. These mutants can be identified as colonies which give an increased response to anti-HSA anti-serum in a direct colony immunoassay. Western blot assays confirm that at least some of the mutants accumulate increased levels of both extracellular and intracellular protein. One strain demonstrating higher levels of protein (an approximate five- to ten-fold increase over the level obtained with the parent strain) has been designated GX5528 and deposited with the American Type Culture Collection, Rockville, MD as ATCC No. 53444.

The GX5528 mutant strain has been found to have acquired an additional auxotrophic marker, a requirement for histidine. A culture of the strain was plated on a minimal agar medium lacking histidine. One of the His+ colonies that grew, a probable revertant, was found to accumulate approximately four times more HSA than does GX5528. This strain has been designated GX5552 and deposited with the American Type Culture Collection as ATCC No. 53442.

The following examples are provided to illustrate the present invention and are not to be construed as limiting.

Example I

Manipulation of **hsa** for Expression

The HSA gene sequence encoding mature HSA can be modified to contain a useful restriction enzyme recognition sequence as follows:

pGX3I5 is a 4 Kb, multicopy E. coli plasmid into which the HSA gene was cloned. The construction of pGX3I5 is described in Figure I.

Plasmid pUC9 is a commercially available (BRL, Rockville, Maryland U.S.A.) plasmid, which is a derivative of pBR322 and contains numerous cloning sites. A multi-site linker fragment was excised from plasmid pUC9 by digestion with EcoRI and HindIII, and inserted into plasmid pGXI26 to form plasmid pGXI33. pGXI26 is a plasmid highly similar to pKGI8I6, differing from it only in the region between the EcoRI and HindIII sites. (pKGI8I6 is described in McKenney et al., A System to Study Promoter and Terminator Signals Recognized by "E.coli" RNA Polymerase, In: Gene Amplification and Analysis, ed. J.G. Cherikjian and T.S. Papas, Elsvier-North Holland [I98I]). pGX3I5 was constructed by deleting the small EcoRI fragment from pGXI33 (see Fig. I).

The HSA gene was modified for insertion into pGX3I5 by replacing a HindIII site in the noncoding region with an EcoRI site. Plasmid pGXI042 (containing a pro-human serum albumin gene, and found within a strain deposited as NRRL No. B-I5783 with Northern Regional Research Laboratories, Peoria, Illinois, U.S.A.) was digested with HindIII, and the linearized DNA was treated with the Klenow fragment of DNA polymerase I. The DNA was ligated to EcoRI linkers, and the ligation mix was digested with HindIII (to reduce the background of transformants from "parental" pGXI042). The treated ligation mix was used to transform E. coli HBI0I (hsd-20 recAI3 ara-I4 proA2 lacYI galK2 rpsL20 xyl-5 mtl-I supE44 [Bolivar and Backman, Methods Enzymol. 68:245-280 [I979]) to ampicillin resistance (see Fig. 2). Plasmid DNA was prepared (Holmes and Quigley, Anal. Biochem. II4:I93-I97 [I98I]) from three of the transformants, and two of the transformants appeared to contain plasmids in which an EcoRI site replaced the HindIII site of pGXI042. This was indicated by the gel electrophoretic patterns obtained upon EcoRI digestion and HindIII+BglI double digestion. One of these plasmids was designated pGX2448.

The modified HSA gene then was subcloned into pGX3I5. pGX2448 and pGX3I5 each were digested with EcoRI+BamHI, and the digests were incubated in the presence of T4 DNA ligase. The ligation mix was digested with SmaI to reduce the frequency of transformants due to pGX2448 and pGX3I5. The hsa-containing subclone was identified among HBI0I transformants by an initial analysis of EcoRI digests of plasmid DNA and was further characterized by a PstI digest and the following double digests: XbaI+EcoRI, PvuII+BglI, and EcoRI+SalI. The pGX3I5 derivative containing hsa was designated pGX2449 (see Fig. 2).

The strategy was to later fuse hsa with amy and npr via a HindIII site (to be inserted into hsa, see below). It was desirable that the HindIII site be unique within the hsa plasmid. To eliminate the extraneous HindIII site present in pGX2449, pGX2449 was digested with HindIII, treated with the Klenow fragment of DNA polymerase I, and ligated to ClaI linkers (CATCGATG) (Fig. 3). The ligation mix was used to transform E. coli strain SK2267 (F-gal thi T1r hstR4 recA endA sbcBI5 [E. coli Genetic Stock Center, Yale University, New Haven, Connecticut]). Plasmids from transformants were initially characterized by a ClaI+BglI double digest and further characterized by gel electrophoresis of HindIII, ClaI, and ClaI+BglII digests. A plasmid lacking the HindIII site was designated pGX245I.

To then insert a HindIII site which could be used to facilitate gene fusion, pGX245I was digested with XhoI and annealed with the partially self-complementary oligonucleotide 5′ TCGAGAAGCTTC, which contains a HindIII recognition sequence. The DNA was ligated, and the ligation mix was used to transform HBI0I. Plasmids from the transformants were characterized initially by electrophoresis of HindIII+BglI digest and subsequently by electrophoresis of a ClaI+PvuII double digest and BglI, BglII, HindIII, PstI, SalI, and XhoI single digests. The plasmid containing a HindIII site was designated pGX2453 (see Fig. 3).

Plasmid pGX2453 was modified to construct a plasmid, pGX246I (see Fig. 3), that can replicate in B. subtilis. pGX2453 was digested with EcoRV, and the digest was ligated to the ClaI fragment of plasmid pEI94 (Horinouchi, S. and B. Weisblum, J. Bacteriology, I50:804-8I4 [I982]). As shown in Figure 3, this fragment was obtained from a plasmid designated pGX329 by digestion with ClaI and treatment with the Klenow fragment of DNA polymerase I to provide two blunt-ended DNA segments. The ligation mix was used to transform E. coli strain GXII70 to ampicillin resistance. Plasmids from the transformants were characterized by ClaI, HincII, PvuII, PstI, HindIII and EcoRI digestion.

Example II

Construction of Plasmids Containing α-Amylase Gene Segments

Segments of amy[BamP] were subcloned to facilitate the construction of gene fusions specifying fusion proteins containing the α-amylase signal sequence joined to HSA sequences. An α-amylase gene was cloned from Bacillus amyloliquefaciens ATCC 23844, generating plasmid pGX2509 (Saunders et al., in Protein Transport and Secretion, pp. 329-339, D. Oxender, ed., Alan R. Liss, Inc., 1984). To make a plasmid that contains a segment of amy[BamP] and also replicates in E. coli, an amy [BamP] segment from GX2509 was sub-cloned into pGX2403.

pGX2403, which contains an erythromycin resistance determinant, ermC, was constructed in two steps from pGX315 (see Example I). First, pGX315 was modified by the insertion of ClaI linkers into the unique SmaI site, generating a plasmid designated pGX333. A ClaI/BamHI fragment containing ermC from pBD9 (Gryczan and Dubnau, PNAS 75: 1428-1432 [1978]) then was inserted between the ClaI and BamHI sites of pGX333, generating pGX2403.

pGX2509 and pGX2403 were each digested with ClaI and EcoRI, and the fragments were ligated together. The ligation mix was used to transform HB101 (Figure 4). Plasmids from the resulting ampicillin resistant transformants were characterized for the presence of two EcoRV sites. A recombinant plasmid, designated pGX2438, was characterized by further restriction digestion with EcoRI+BamHI, ClaI+PstI, and PvuI.

pGX2438 then was modified to allow its integration into the B. subtilis chromosome, although this modification was not utilized in the following experiments. A BglI+HindIII fragment from pGX281 (Saunders et al., J. Bacteriol. 157: 718-726 [1984]) containing a 1 kb segment of the B. subtilis chromosome was used to replace a corresponding BglI+HindIII fragment from pGX2438 (Figure 4). BglI+HindIII digests of the two plasmids were ligated and used to transform HB101 to ampicillin resistance. The resulting transformants were screened by well lysis (Eckhardt, Plasmid 1:584-588 [1978]) for plasmids of the appropriate size. In addition, transformants were screened for growth on medium containing 500 μg/ml erythromycin to detect the presence of ermC. The plasmid chimera, pGX2442, then was characterized by restriction digestion with HindIII, BglII+HindIII, EcoRI+EcoRV, ClaI+PstI, and BamHI+HindIII.

The unique HindIII site of pGX2442 then was removed to facilitate further manipulations (Figure 4). After HindIII digestion, pGX2442 was treated with the Klenow fragment of DNA polymerase I in the presence of four deoxyribonucleotide triphosphates. The DNA was ligated, and the ligation mix was used to transform HB101. A plasmid, pGX2443, from one of the resulting transformants had lost the HindIII site. It was further characterized by other restriction enzyme digestions, including ClaI+HindIII, EcoRI, and BglI.

amy gene segments of different lengths were generated and identified by fusing them to a segment of the pBR322-encoded β-lactamase gene, bla. A plasmid, pKTH74, containing a bla gene segment encoding the mature β-lactamase but not its signal sequence was obtained from I. Palva (Palva et al., PNAS 79: 5582-5586 [1982]). The bla segment is preceded by a HindIII site that can be used for gene fusions. It was reasoned that only α-amylase gene segments that specified protein export signals and were in the appropriate reading frame relative to bla would direct β-lactamase secretion and, therefore, ampicillin resistance in E. coli (Kadonaga et al., J. Biol. Chem. 260:16192-16199 [1985]).

pGX2443 was digested with EcoRI and treated with nuclease BAL-31 to generate fragments of various lengths. The DNA was ligated to HindIII linkers, and the ligation mix was treated with BglI (Figure 5). The smaller HindIII fragment of pKTH74 was gel purified and digested with BglI. The DNAs were ligated, and the ligation products were used to transform HB101 to ampicillin resistance. Many transformants were recovered and shown to contain plasmids with different lengths of amy. However, only two amy segments were characterized by DNA sequencing. pGX2472 contains the HindIII linker immediately adjacent to the coding region for the carboxy terminus of the signal sequence (Figure 6). pGX2473 is similar, but it also encodes three amino acids of mature α-amylase preceding the HindIII linkers.

To generate a derivative of pGX2472 that would replicate as a plasmid in B. subtilis, a two-step procedure was used (Figure 7). pGX312 is a recombinant plasmid containing pUB110 as a BamHI fragment (Saunders et al., J. Bacteriol. 157: 718-726 [1984]). pGX312 was digested with BamHI and XhoI (to inactivate one of the two BamHI fragments), and the restriction fragments were ligated to BamHI-digested pGX2472. The ligation mix was used to transform HB101 to ampicillin resistance with screening on agar medium containing 10 μg/ml kanamycin. A transformant was found that contained the desired plasmid, pGX2446, as indicated by restriction enzyme analysis using EcoRI, BamHI, BglI, PstI, PvuI, PvuII, XbaI, SalI, and EcoRI+SalI.

pGX2446 contained a direct repeat of about 300 bp of pUB110 which was removed (see Figure 7). pGX2446 was digested with EcoRI and SalI, and the restriction fragments were treated with the Klenow fragment of DNA polymerase I in the presence of the four deoxyribonucleotide triphosphates. The DNA was ligated, and the ligation mix was used to transform HB101. pGX2447 was found in one of the transformants. The plasmid was characterized by digestion with XbaI, BamHI, SalI, EcoRI, HindIII, PstI, and BamHI.

## Example III

### Fusion of **amy** to **hsa**

Gene fusions (ahs) containing amy and hsa were constructed and maintained in B. subtilis on either plasmid or chromosomal replicons. To generate the plasmid-borne ahs-1 fusion, pGX2447 was digested with ClaI+HindIII+BglI and ligated to pGX2461 that had been digested with ClaI+HindIII (Figure 7). The ligation mixture was used to transform competent BR151 cells (Saunders et al., J. Bacteriol. 157: 718-726 [1984]). Erythromycin resistant transformants were scored for the production of HSA cross-reacting material (CRM) production by a direct colony immunoassay.

To perform the direct colony immunoassay, a cell sample was streaked on a membrane filter "sandwich" which had been placed on the surface of a tryptose blood agar base medium containing 50 µg/ml erythromycin. The "sandwich" was prepared by first placing a nitrocellulose membrane (Schleicher and Schuell, catalog number BA85) on top of the agar medium and then placing a cellulose acetate membrane (Schleicher and Schuell, catalog number OE67) on top of that. The inoculated plate was incubated overnight at 37° C. The nitrocellulose membrane then was removed and developed immunochemically. It was first incubated in a blocking solution (TS buffer [10 mM Tris Hydrochloride pH 8.05 0.9% sodium chloride] containing 3% Knox® gelatin) for at least 5 minutes. It then was incubated in a primary antibody solution of a mouse monoclonal antibody [CL 2513-A; Cedarlane Laboratories Limited; Hornby, Ontario, Canada]. The antibody was diluted in TS buffer containing 1% gelatin. The filter was incubated with the primary antibody for 1-18 hours at room temperature. It then was washed three times with TS buffer and incubated with a secondary anti-mouse IgG antibody-horseradish peroxidase (HRP) conjugate. The incubation with secondary antibody was also at least one hour at room temperature. The presence of HSA CRM was inferred from the presence of the bound HRP-coupled antibody and detected using the following developing solution:

| | |
|---|---|
| 4-chloro-1-naphthol | 36 mg |
| methanol | 12 ml |
| TS buffer | 48 ml |
| 30% $H_2O_2$ | 120 µl |

This was prepared by adding the components in the order listed and was used immediately. After development of the purple color indicating the presence of HRP, the filters were rinsed with water and air-dried.

One CRM positive transformant was found to contain a plasmid designated pGX2464 (Figure 7). The nature of the plasmid was established by restriction enzyme analysis with PstI, HindIII + PstI, and XbaI. This transformant has been designated GX7003.

The construction of a pUB110-derived vector containing ahs-1 was performed in two steps. Initially, pGX2446 was digested with EcoRI and SalI. The digest was treated with the Klenow fragment of DNA polymerase I in the presence of four deoxyribonucleotide triphosphates. The DNA was ligated in the presence of EcoRI linkers, and the ligation mix was used to transform GX1170. Among the transformants was found a strain containing a plasmid, pGX2462, in which an EcoRI site had replaced the region from the EcoRI site to the SalI site in pGX2446.

pX2462 and pGX2453 were each digested with EcoRI and HindIII, and the digested DNA was ligated. The ligation mix was used to transform BR151 cells to erythromycin resistance. Erythromycin resistant colonies were scored for HSA CRM production by the direct colony immunoassay. One HSA CRM-producing isolate contained a plasmid, pGX2465, which conferred both erythromycin and kanamycin resistance and contained ahs-1.

A plasmid-borne ahs-2 gene fusion was constructed by inserting the ClaI + HindIII fragment containing amy from pGX2473 between the ClaI and HindIII sites of pGX2461. The resulting plasmid was designated pGX1812, and a BR151 host transformed with this plasmid has been designated GX4129.

To generate a chromosomally carried ahs-1 fusion, pGX2473 and pGX2453 were each digested with HindIII and BamHI, and the restriction fragments were ligated. The ligation mix was used to transform competent B. subtilis BR151 cells (Figure 8). Erythromycin resistant transformants were recovered and tested for HSA CRM production by the direct colony immunoassay described above. DNA from one of the HSA CRM-positive transformants, GX7073, was used to transform IS53 (spoOA677 [Bacillus Genetic Stock Center, Ohio State University, Columbus, Ohio]) to chloramphenical resistance generating a strain, GX7074, which synthesizes HSA CRM.

An ahs fusion, ahs-3, specifying the α-amylase signal sequence and HSA but no additional amino acids (Fig. 11), also was generated. This was done by sub-cloning a segment of ahs-1 into M13mp9, performing oligonucleotide-directed mutagenesis (as described by Zoller and Smith, Nucleic Acid Res. 10:6487-6500 [1982]), confirming the presence of the mutation by DNA sequencing (Sanger et al., PNAS USA 74:5463-5467 [1977]) and sub-cloning the mutagenized fragment into pGX2461. The resulting plasmid was designated pGX2468 and a BR151 transformant harboring the plasmid has been designated GX7034.

A series of other hsa expression vectors was derived from a derivative of pGX2464. To facilitate oligonucleotide-directed mutagenesis, a 1 kb fragment containing the origin of replication of the filamentous coliphage f1 was inserted into the unique EcoRI site of pGX2464 (Figure 9). The resulting plasmids, designated pGX3804 and pGX3805, can replicate to produce single-stranded DNA in E. coli if the host strain is superinfected with a related phage IR1 (Schmidt et al., Gene, in press). Similarly, a plasmid was constructed which was a derivative of pGX2461 in which a 1 kb fragment containing the f1 origin of replication was inserted. The resulting plasmid was designated pGX3803. An E. coli strain designated GX7028, which contains pGX3805, has been deposited with the ATCC as No. 39993. An E. coli strain designated GX7031, which contains pGX3804, has been deposited with the ATCC as No. 39995.

In one example, the single-stranded version of pGX3805 was used as a template for oligonucleotide-directed mutagenesis (Schmidt, et al., Gene, in press) to generate a new ahs (ahs-4) fusion in which the coding sequence for the amylase signal sequence is adjacent to the coding sequence for pro-HSA (Figures 10, 11). Note that ahs-3 (see above) and ahs-4 specify, in addition to the amino acids of the α-amylase signal sequence, only HSA and proHSA amino acids, respectively. Neither specifies linker-encoded amino acids.

The plasmid (pGX3804) containing the ahs-1 fusion also was modified by placing a putative transcriptional terminator adjacent to hsa (promoter distal) to determine if such a transcriptional terminator might enhance hsa expression. This required a two-step approach to generate the appropriate plasmid. First, a SalI site was introduced into pGX3804, very near the sequence encoding the translational termination codon of hsa. This was done using oligonucleotide-directed mutagenesis (Figure 9). The resulting plasmid, pGX3816, was characterized by restriction nuclease analysis with SalI+XhoI, HincII, SalI,PstI, and SalI+ClaI. An oligonucleotide designed to be a transcriptional terminator then was inserted into the SalI site. The inserted sequence bears considerable homology to the presumed transcriptional terminator following a neutral protease gene from B. amyloliquefaciens strain P (Vasantha et al., J. Bacteriol. 159: 811-819 [1984]); the difference between the two is the presence of an EcoRV site in the "loop" region of the synthetic sequence. pGX3816 was digested with SalI, and the linearized vector was ligated to the synthetic oligonucleotide. The ligation mix was used to transform GX1210 (F′ traD36 proA+B+ lacIq/ ΔlacZm15) to ampicillin resistance. One of the resulting transformants carried a plasmid, pGX3821, which contained the inserted sequence. The plasmid was characterized by restriction enzyme analysis with SalI+EcoRV, XhoI+EcoRV, PstI, EcoRV, SalI+EcoRI, and XbaI. Further, DNA sequencing established that the sequence had indeed been inserted as designed. A B. subtilis IS53 strain transformed by this vector has been designated GX7097.

Finally, to provide an appropriate negative control, the ClaI+HindIII amy promoter and signal sequence coding fragment from pGX3821 was replaced with the corresponding promoterless fragment from pGX2461, generating pGX3832.

Example IV

Fusions of **npr** to **hsa**

Vasantha et al. J. Bacteriol, 1986, in press) have described a vector, pGX2134, containing a segment of a neutral protease gene, npr[BamP], from B. amyloliquefaciens. The npr segment comprises the promoter, ribosomal binding site, translation initiation codon and nucleotide sequence for the entire coding region of the signal sequence. It contains a BamHI site at the sequence specifying what appears to be the second amino acid of the "pro" region (Vasantha et al., J. Bacteriol., 1986, in press). This vector was further modified by the insertion, at the BamHI site, of an oligonucleotide specifying a HindIII site (Figure 12), generating a plasmid designated pGX4509. pGX4509 was then modified by insertion of a ClaI linker at the unique EcoRI site, generating pGX3834. A ClaI+HindIII digest of pGX3834 then was ligated to a ClaI+HindIII digest of pGX3832. The ligation mixture was used to transform competent BR151 cells to erythromycin resistance. A B. subtilis strain synthesizing HSA CRM was identified by the direct colony immunoassay (see Example III), purified, and found to contain a recombinant plasmid, designated pGX3837, which was characterized by restriction enzyme digestion with XbaI+PvuI, BglII, and EcoRI. B. subtilis host strain IS53 transformed with pGX3837 has been deposited with the American Type Culture Collection (ATTC), Rockville, Maryland as No. 53443.

To generate a strain containing npr/hsa in the chromosome, pGX3837 and pGX281 (Saunders et al., J. Bacteriol. 157:718-726 [1984]) were digested with EcoRI and ligated. The ligation mix was used to transform B. subtilis BR151 competent cells to chloramphenicol resistance. Transformants producing HSA CRM were identified by the direct colony immunoassay. Several HSA CRM-producing isolates were picked, and DNA from one of them (GX7074) was used to transform competent IS53 cells to chloramphenicol resistance, generating strain GX7078.

Example V

Chemical Mutagenesis to Produce **B. subtilis** Strains That Produce More HSA CRM

To generate B. subtilis strains producing higher levels of HSA CRM, nitrosoguanidine mutagenesis of GX7002 was used. GX7002 is derivative of GX4937 (trp-2 lys-3 metB10 spoOA677,apr::cat,Npr-) containing pGX3804 (Figure 9). Following nitrosoguanidine treatment, the culture was plated, and individual colonies were analyzed by the direct colony immunoassay. Among several isolates that produced a more intense reaction with the HSA antibody, GX5528 was further characterized. It was found to also be His-, and a His+ revertant, GX5552, was isolated as a colony which was able to grow on minimal medium in the absence of histidine.

Example VI

The Production and Secretion of HSA CRM

To test the production and secretion of HSA CRM from GX8006 (a nitrosoguanidine mutagenized derivative of GX7002, which contains ahs-1), Western blot analysis was performed. GX8006 was cultured in 10 ml of 2% yeast extract (Difco) containing 50 µg/ml erythromycin for 24 hours at 37°C. Cell and supernatant fractions were prepared as follows. The culture was centrifuged for 10 minutes at 5,000 rpm in a GLC centrifuge, A500 rotor, at room temperature. The supernatant was removed, and phenylmethyl sulfonyl fluoride (PMSF) and $Na_2$EDTA (disodium ethylene diamine tetraacetate) were added to give final concentrations of 1 mM and 5 mM, respectively. To remove any contaminating cells from the supernatant fraction, the supernatant was centrifuged for 15 minutes at 15,000 rpm in a SM24 rotor at 4°.

Samples of the supernatant fraction were then processed by each of two methods. For trichloracetic acid (TCA) precipitation, 1 ml of supernatant was mixed with 111 µl of 100% TCA. After incubation of the samples for one hour on ice, the precipitate was collected by spinning 15 minutes at 4° in a microfuge. The precipitates were washed with 500 µl of 5% TCA and resuspended in 50 µl of sample prep (SPB) buffer (50 mM Tris-hydrochloride [pH 6.8], 1% SDS, 2 mM disodium EDTA, 10% glycerol) also containing 5% β-mercaptoethanol (BME), 5 mM $Na_2$EDTA, 1 mM PMSF, and 40 mM Tris base. After vortexing for one minute and boiling for 5 minutes, the samples were kept on ice.

For concentration using Centricon 30 (Amicon) filtration, 2 ml of supernatant were concentrated as directed by the manufacturer. The concentrates were washed twice with TSPE buffer (10 mM Tris hydrochloride pH 8.0; 0.9% sodium chloride, 5 mM $Na_2EDTA$, and 1 mM PMSF). The samples were mixed with an equivalent volume of 2 × SPB (containing 10% BME, 1 mM PMSF, and 5 mM $Na_2EDTA$). The final sample represented a 15-fold concentration of the culture supernatant.

The cell fractions were washed twice with 5 ml of TPEC buffer (55 mM Tris hydrochloride, pH 8.0; 5.5 mM $Na_2EDTA$, 1.1 mM PMSF, 22 μg/ml chloramphenicol) and resuspended in 550 μl of TPEC. A portion (100 μl) of the cell pellet was mixed with 10 μl of lysozyme (20 mg/ml) and incubated at 37 degrees for 50 minutes. Tween-20 (10 μl) and 1 volume of 2 x SPB (containing 10% BME, 1 mM PMSF, and 5 mM EDTA) were added, and the sample was boiled for 5 minutes.

Samples were electrophoresed through a 10% polyacrylamide gel, with a 5% polyacrylamide stacking gel, as described by Studier (J. Mol. Biol. 79:237-248 [1973]). The cell sample was equivalent to 40 μl of culture, and the supernatant samples were each equivalent to 250 μl of culture.

The proteins were electroblotted (Bers and Gartin, Biotechniques, 3:276-288, [1985]) from the gel to nitrocellulose (Schleicher and Schuell) and developed for antigen as described for the direct colony immunoassay (see Example III).

As shown in Figure 13, HSA CRM was found in both the cell and supernatant fractions; and no such immunoreactive material was found in samples from a negative control strain, GX5523 (not shown, but see below). (GX5523 is host strain GX4937 transformed with a pGX2461 [depicted in Figure 3]). In the same experiment, the HSA CRM detected from the cell and supernatant fractions of GX5528 produced a similar pattern.

In Figure 13, Lane A represents the cell sample: Lane B, the supernatant sample which was TCA concentrated; Lane C, the supernatant sample which was Centricon concentrated; Lane D, HSA (1 ug) (Sigma Chemical Company) mixed with supernatant from GX5523, a negative control strain. This mixture sample was also prepared in 1 × SPB.

The most prominent species of HSA CRM in the supernatant fraction comigrated with purified HSA purchased from Sigma Chemical Company. In contrast, the most prominent species of HSA CRM in the cell fraction had a lower gel electrophoretic mobility, which suggested a molecular weight of 71 KD, consistent with its being HSA CRM with the amylase signal sequence attached. The different gel electrophoretic mobilities of the prominent bands of HSA CRM in the cell and supernatant fractions provide strong evidence that the presence of HSA CRM in the supernatant was a result of secretion through the B. subtilis membrane and not the result of cell lysis. This interpretation was substantiated by further work (see below).

A separate experiment provided further evidence that the extracellular HSA CRM was not a result of cell lysis. The proteins from a culture grown for 24 hours in 2% yeast extract containing 10 μg/ml erythromycin was examined (data not shown). Cell and supernatant samples were analyzed by electrophoresis through polyacrylamide gels which subsequently were stained with Coomassie blue. Most of the proteins represented in the cell fraction apparently were not present in the supernatant fraction, indicating that cell lysis was minimal. Equivalent cell and supernatant fractions were analyzed by immuno blotting (Bers and Gertin, BioTechniques 3:276-288 [1985]) using antisera against HSA. More than 1/2 of the HSA CRM was in the culture supernatant. Therefore, the HSA CRM was being preferentially accumulated in the culture supernatant, unlike most (or all) cellular proteins. The different electrophoretic mobility of cell-associated and supernatant HSA CRM and the absence of evidence for cell lysis in these cultures together indicates that the extracellular HSA was a product of secretion through the membrane.

In addition to the above experiments, the production of HSA CRM was assayed by immunoprecipitation of pulse-labeled cells. Four strains were grown in S7 medium (Saunders et al., J. Bacteriol. 157: 718-726 [1984]) to optical densities at 600 nm (Bausch and Lomb Spectrophotometer 20) ranging from 0.4 to 0.7. The strains were GX7076 (B. subtilis IS53 transformed with pGX3812 [Figure 10]), GX7075 (IS53 transformed with pGX3805 [Figure 9]), GX7091 (IS53 transformed with pGX2465) and GX7093 (IS53 transformed with pGX3803 [Example III]). Part (1 ml) of each culture was incubated in the presence of $^{35}S$ cysteine (New England Nuclear) for 10 minutes. The radioactive labeling was terminated by the addition of unlabeled cysteine, chloramphenicol, casamino acids, $Na_2EDTA$, and PMSF to give final concentrations of 50 μg/ml, 50 μg/ml, 0.2%, 5 mM, and 1 mM, respectively. Lysozyme (50 ul of 20 mg/ml) was added, and the samples were incubated at room temperature for 3 minutes. TCA was added to give a final concentration of 8%, and the samples were incubated overnight at 4°C.

Samples were immunoprecipitated as described by Saunders et al., J. Bacteriol. 157:718-726 (1984), except that 2 μl of antiserum to HSA (Calbiochem) were used. Samples were electrophoresed through a 7.5% polyacrylamide gel (Laemmli, Nature 227:680-685 [1970]) at 40 milliamps. The gel was soaked in autofluor (National Diagnostics), dried, and exposed to Kodak XAR-2 film at -80° degrees.

As shown in Figure 14, proteins of about 68,000 kd were isolated from GX7076 (ahs-4) (Lane C), GX7091 (ahs-1) (Lane A), and GX7075 (ahs-1) (Lane D). In contrast, there was no such material immunoprecipitated from a negative control strain, GX7093 (Lane B). The gel electrophoretic mobility of the HSA CRM was slightly different for each of the three HSA CRM-producing species shown in Figure 14. The HSA CRM isolated from GX7075 migrated as a doublet. In contrast, only a single species of HSA CRM was isolated from GX7076, and it corresponded to the upper band of the GX7075 sample doublet. Only a species of HSA CRM corresponding to the lower band of the GX7075 sample doublet was isolated from pGX7091. As described below, it is theorized that the species of HSA CRM found in the lower band is HSA CRM from which the signal sequence has been removed, as was the case in the immunoblot experiment described above. Therefore, it appears that the signal sequence is not removed from the ahs-4 product.

Further experiments were conducted using BRI5I derivative strains. BRI5I derivatives were assayed for HSA CRM production by immunoprecipitation. The strains were GX7033 (B. subtilis strain BRI5I transformed with pGX38I2 [Figure I0]), GX7032 (BRI5I transformed with pGX3805 [Figure 9]), GX7034 (BRI5I transformed with pGX2468 [Example III]) and GX7003 (BRI5I transformed with pGX2464 [Figure 7]). Cultures were grown as described in the preceding paragraph, with the exception that 50 µg/ml each of tryptophan, lysine, and methionine were added to the S7 to meet the auxotrophic requirements of BRI5I derivatives. The cultures were pulse labeled with $^{35}$S cysteine for I0 minutes and the samples immunoprecipitated and treated as described above. As shown in Figure I5, upon electrophoresis of HSA CRM immunoprecipitated from the derivatives, the same pattern of a single, upper band of HSA CRM was found for an ahs-4 carrying strain, GX7033 (lane A). There was again a doublet isolated from an ahs-I carrying strain, GX7032 (Lane B). In addition, there was a doublet isolated from an ahs-3 carrying strain, GX7034 (Lane C).

As a control that the immunoprecipitable proteins were HSA CRM, there was a similar doublet produced by GX7003 (ahs-I) which was observed following immunoprecipitation using rabbit antiserum to HSA (Calbiochem), whereas no such protein was detected following immunoprecipitation using a control rabbit antiserum (not shown).

To further investigate the nature of the bands of HSA CRM, phenylethyl alcohol (PEA) treatment of GX7075 was used. In PEA treated bacteria, signal sequence removal and secretion are inhibited (Daniels et al., PNAS USA 78:5396-5400 [I984]).

Three cultures of GX7075 were grown in S7 medium, and PEA was added to two of the cultures to give a final concentration of 0.2% in one culture and 0.4% in the second culture. The cultures were incubated in the presence of PEA for I0 minutes and then pulse labeled as described previously. Samples were immunoprecipitated and analyzed by electrophoresis through 7.5% polyacrylamide gels. The aforementioned doublet was observed in the absence of PEA (Figure I6, lane B), but only the upper band was observed in the presence of the PEA (Figure I6, lanes C, D). Therefore, there is a correlation between inhibition of signal sequence removal and inhibition of the appearance of the lower band of the HSA CRM. In Figure I6, lane A represents the GX7076 (no PEA) sample; lane B, GX7075 (no PEA) sample; lane C, GX7075 (0.2% PEA) sample; and lane D, GX7075 (0.4% PEA) sample.

The nature of the upper and lower bands of HSA CRM also may be identified through the correlation of these species with secretion from pulse labeled cells. Unlike cultures grown for 24 hours in 2% yeast extract, only a very small fraction of the HSA CRM was secreted from pulse-labeled cells, even with a 2 hour chase after pulse labeling. However, HSA CRM was secreted from lysozyme-generated protoplasts (Figure I7). GX7075 and GX7076 were grown in S7 medium, and part (2 ml) of the cultures was diluted by adding an equal volume of I M sucrose, dissolved in S7. Lysozyme (I50 ul of 2 mg/ml) was added,and the cultures were incubated for 90 minutes at 37°C. The protoplasts were pelleted by centrifuging for 7 minutes at 4,000 rpm at room temperature. The protoplasts were resuspended in 0.5 M sucrose, in S7 and pulse labeled with $^{35}$S cysteine for I0 minutes, transferred to a microfuge tube containing I0 ul of 0.5 M $Na_2$EDTA and I0 µl of 0.I M PMSF. The samples were spun for 2 minutes at room temperature in a Fisher microfuge. The supernatant was transferred to a microfuge tube containing I0 µl of 0.5 M $Na_2$EDTA, 2 µl of 0.I M PMSF, and I00 µl of I00% TCA. The protoplast pellet was resuspended in I ml of 0.5M sucrose, in S7. EDTA, PMSF, and TCA were then added to give final concentrations of 5 mM, 0.2 mM, and 8%, respectively. The samples were immunoprecipitated and treated as described above for pulse-labeled cells. In Figure I7, lane A represents the HSA CRM isolated from the GX7075 cell fraction; lane B the GX7075 supernatant, lane C the GX7076 cell fraction and lane D the GX7076 supernatant. For a given strain, the efficiency of secretion from protoplasts correlated very well with the appearance of the lower band of HSA CRM. Protoplasts of GX7075, which carry ahs-I, secreted over I/2 of the HSA CRM to the culture supernatant, while protoplasts of GX7076, which carry ahs-4, did not secrete significant amounts of the HSA CRM to the culture supernatant.

On the basis of the above experiments, gel electrophoretic mobility can be used as a criterion for signal sequence removal from HSA CRM. As judged by gel electrophoretic mobility and, in most cases, secretion from protoplasts, an inverse correlation was found in pulse-labeling experiments between the fraction of HSA CRM from which the signal sequence was removed and the level of hsa expression. This was best demonstrated with a variety of strains containing ahs-I. As described above, about I/2 of the HSA CRM had the signal sequence removed from GX7075 (Figure I3). In contrast, only HSA CRM from which the signal sequence had been removed was observed with GX709I, which produced only about I/2 as much HSA CRM (as a fraction of total protein synthesis) in a pulse-labeling experiment. GX7074, which contains ahs-I integrated into the B. subtilis chromosome, produces much less HSA CRM than does GX7076, but essentially all of the HSA CRM has had its signal sequence removed (not shown). A similar pattern existed for npr/hsa. HSA CRM with a signal sequence attached was detected upon analysis of GX7080, B. subtilis strain IS53 transformed with plasmid-borne npr/hsa. However, mostly HSA CRM from which the signal sequence had been removed was observed upon analysis of GX7078, (host IS53 containing chromosomal npr/hsa) which synthesizes much less HSA CRM. Therefore, HSA CRM is efficiently secreted from B. subtilis at relatively low levels of hsa expression. However, at higher levels of hsa expression, the signal sequence is not efficiently removed.

Example VII

**B. subtilis** Strains That Overproduce HSA CRM

GX5528 was identified by its ability to produce a more intense reaction than GX7002 in the direct colony immunoassay (see Example IV). The HSA CRM accumulated by GX5552 was analyzed by Western blot analysis. It was found that GX5552 accumulated more HSA CRM by this assay as well (Figure I8). For this experiment. GX5528 and GX7002 were cultured in 2% yeast extract containing 50 µg/ml of erythromycin for 24 hours at 37.C. Samples were prepared as described in the first procedure set forth in Example VI. In Figure I8, Lane A represents the GX7002 cell sample, Lane B represents the GX7002 supernatant sample which was TCA concentrated, Lane C represents the GX7002 supernatant sample which was centricol concentrated, Lane D represents the GX5528 cell sample and Lane E represents the GX5528 supernatant sample which had been TCA concentrated. The mutation responsible for the HSA overproduction phenotype was chromosomally borne, as indicated by experiments involving curing the plasmid from GX5528, transforming the cured derivative with pGX3804, and assaying the resulting transformants (data not shown). In addition, the plasmid from pGX5528 was used to transform GX4937 to erythromycin resistance. The resulting transformants produced the less intense reaction, characteristic of GX7082, on the direct colony immunoassay. Therefore, it is possible to generate, by nitrosoguanidine mutagenesis, strains that overproduce HSA CRM without having suffered mutations in the HSA gene.

Another method for effecting overproduction of HSA involves a putative transcriptional terminator. A BRI5I derivative carrying pGX382I produced about 2-fold more HSA CRM (as a fraction of total protein synthesis) in a pulse labeling (as described above) than did a BRI5I derivative carrying pGX3804. As the only known difference between the two strains in the presence of the putative transcriptional terminator following hsa in pGX382I, this sequence appears to be responsible for enhanced hsa expression.

**Claims**

I. A gene fusion comprising a nucleotide sequence (hsa) encoding human serum albumin (HSA) fused in correct reading frame, either directly or via a linker sequence, to the expression elements and signal sequence coding region of a gene that can be recognized by the transcription and translation systems of a Bacillus host, such that the expression elements of said gene control the expression of said HSA-encoding sequence.

2. A gene fusion as claimed in claim I, wherein the expression elements and signal sequence coding region comprise a segment of the α-amylase gene (amy[BamP]) from Bacillus amyloliquefaciens and the Bacillus host is B. subtilis.

3. A gene fusion as claimed in claim 2, wherein the segment of amy[BamP] gene comprises a promoter, a ribosome binding site, a translation initiation codon, and a nucleotide sequence coding for the entire signal sequence, and is bounded at its carboxy terminus coding region by a synthetic linker to which is fused the hsa nucleotide sequence.

4. A gene fusion as claimed in claim 2, wherein the segment of amy[BamP] gene comprises a promoter, a ribosome binding site, a translation initiation codon, a nucleotide sequence coding for the entire signal sequence and a nucleotide sequence that specifies the three amino-terminal amino acids of extracellular α-amylase, and is bounded at the carboxy terminus coding region by a synthetic linker to which is fused the hsa nucleotide sequence.

5. A gene fusion as claimed in claim 3 or claim 4, wherein said synthetic linker comprises a HindIII synthetic linker.

6. A gene fusion as claimed in claim 3 or claim 4 which is constructed by ligating said segment of amy[BamP] gene to said hsa nucleotide sequence, wherein prior to said ligation said hsa sequence is derived from a preproHSA structural gene, which is modified by means comprising

a) in vitro mutagenesis to create a restriction enzyme recognition site within the pro-coding sequence of said structural gene:

b) digestion with a restriction enzyme which recognizes said site; and

c) ligation to a synthetic oligonucleotide containing a restriction enzyme recognition site which can be cleaved by a restriction enzyme to provide said hsa gene with an amino terminus coding region which is complementary to a synthetic linker at the carboxy terminus coding region of the amy[BamP] gene segment.

7. A gene fusion as claimed in claim 6, wherein said preproHSA structural gene is modified by in vitro mutagenesis to create an XhoI site within the pro-coding sequence, followed by digestion with XhoI and ligation to a synthetic oligonucleotide linker containing a HindIII site; said carboxy terminus coding region of the amy[BamP] gene segment is bounded by a HindIII synthetic linker, and said amy[BamP] gene segment and the modified hsa nucleotide sequence are digested with HindIII prior to ligation.

8. A gene fusion as claimed in claim 7 which is modified by oligonucleotide-directed mutagenesis to delete nucleotides which specify amino acids encoded by said HindIII synthetic linker and a portion of the HSA pro sequence such that the signal sequence coding region of the amy[BamP] gene is fused directly to a nucleotide sequence encoding mature HSA.

9. A gene fusion as claimed in claim 7 which is modified by oligonucleotide-directed mutagenesis such that eight base pairs of the HindIII synthetic linker are deleted and fourteen base pairs are substituted therefore such that the signal sequence coding region of the amy-[BamP] gene is ligated directly to a nucleotide sequence encoding proHSA.

10. A gene fusion as claimed in claim 1, wherein the expression elements and signal sequence coding region comprise a segment of a neutral protease gene (npr[BamP]) from Bacillus amyloliquefaciens and the Bacillus host is B. subtilis.

11. A gene fusion as claimed in claim 10, wherein the segment of npr[BamP] gene comprises a promoter, a ribosome binding site, a translation initiation codon, a nucleotide sequence coding for the entire signal sequence and a nucleotide sequence which specifies the amino-terminal two amino acids of the "pro" region of the protease, and is bounded at its carboxy terminus coding region by a synthetic linker to which is fused the hsa nucleotide sequence.

12. A gene fusion as claimed in claim 11 which is constructed by ligating said segment of npr[BamP] gene to said hsa nucleotide sequence, wherein prior to said ligation said hsa sequence is derived from a preproHSA structural gene, which is modified by means comprising

a) in vitro mutagenesis to create a restriction enzyme recognition site within the pro-coding sequence of said structural gene;

b) digestion with a restriction enzyme which recognizes said site; and

c) ligation to a synthetic oligonucleotide containing a restriction enzyme recognition site which can be cleaved by a restriction enzyme to provide said hsa gene with an amino terminus coding region which is complementary to a synthetic linker at the carboxy terminus coding region of the npr[BamP] gene segment.

13. A gene fusion as claimed in claim 12, wherein said preproHSA structural gene is modified by in vitro mutagenesis to create an XhoI site within the pro-coding sequence, followed by digestion with XhoI and ligation to a synthetic oligonucleotide linker containing a HindIII site; said carboxy terminus coding region of the npr[BamP] gene segment is bounded by a HindIII synthetic linker, and said npr[BamP] gene segment and the modified hsa nucleotide sequence are digested with HindIII prior to ligation.

14. A gene fusion as claimed in any one of claims 1 to 13, wherein said nucleotide sequence encoding HSA comprises DNA encoding a translation stop codon and wherein a transcription terminator is placed near the DNA specifying said translational termination codon, on the promoter-distal side.

15. A plasmid vector comprising a gene fusion as claimed in any one of claims 1 to 14.

16. A plasmid vector as claimed in claim 15, further comprising a functional replicon for said Bacillus host.

17. A plasmid vector as claimed in claim 16 selected from plasmids pGX2464, pGX1812, pGX2468, pGX3804, pGX3805, pGX3821, pGX3812 and pGX3837.

18. A strain of Bacillus transformed by a vector as claimed in any one of claims 15 to 17.

19. A transformed strain as claimed in claim 18 wherein said gene fusion is inserted in the chromosomal DNA.

20. A Bacillus strain having the identifying characteristics of GX7003.

21. A Bacillus strain having the identifying characteristics of GX4129.

22. A Bacillus strain having the identifying characteristics of GX7034.

23. A Bacillus strain having the identifying characteristics of GX7033.

24. A Bacillus strain having the identifying characteristics of GX7097.

25. A Bacillus strain having the identifying characteristics of GX7080 (deposited with the ATCC as 53443).

26. A method for producng a protein having the antigenic characteristics of HSA which comprises cultivating a transformed Bacillus strain as claimed in any one of claims 18 to 25 in a nutrient medium under conditions for the production of said protein.

27. A method as claimed in claim 26 wherein said protein is secreted into said nutrient medium.

28. A method as claimed in claim 27 wherein said nutrient medium comprises about 2% yeast extract.

27. A method as claimed in claim 26 wherein said protein is secreted into said nutrient medium.

28. A method as claimed in claim 27 wherein said nutrient medium comprises about 2% yeast extract.

29. A method as claimed in any one of claims 26 to 28 wherein a transformed Bacillus strain as claimed in any one of claims 18 to 25 is chemically mutagenized prior to being grown in a nutrient medium so as to result in a higher level of production of said protein.

30. A method as claimed in claim 29 wherein said transformed strain is mutagenized with nitrosoguanidine.

31. A strain having the identifying characteristics of GX5528 (deposited with the ATCC as 53444).

32. A strain having the identifying characteristics of GX5552 (deposited with the ATCC as 53442).

33. A process for preparing a gene fusion comprising a nucleotide sequence (hsa) encoding human serum albumin (HSA) and the expression elements and signal sequence coding region of a gene that can be recognized by the transcription and translation systems of a Bacillus host which comprises fusing said hsa nucleotide sequence in the correct reading frame, either directly or via a linker sequence, to said expression elements and signal sequence coding region, such that expression of said hsa nucleotide sequence is under the control of said expression elements.

Claims for the following Contracting State : AT.

1. A process for preparing a gene fusion comprising a nucleotide sequence (hsa) encoding human serum albumin (HSA) and the expression elements and signal sequence coding region of a gene that can be recognized by the transcription and translation systems of a Bacillus host which comprises fusing said hsa nucleotide sequence in the correct reading frame, either directly or via a linker sequence, to said expression elements and signal sequence coding region, such that expression of said hsa nucleotide sequence is under the control of said expression elements.

2. A process as claimed in claim 1, wherein the expression elements and signal sequence coding region comprise a segment of the α-amylase gene (amy[BamP]) from Bacillus amyloliquefaciens and the Bacillus host is B. subtilis.

3. A process as claimed in claim 2, wherein the segment of amy[BamP] gene comprises a promoter, a ribosome binding site, a translation initiation codon, and a nucleotide sequence coding for the entire signal sequence, and is bounded at its carboxy terminus coding region by a synthetic linker to which is fused the hsa nucleotide sequence.

4. A process as claimed in claim 2, wherein the segment of amy[BamP] gene comprises a promoter, a ribosome binding site, a translation initiation codon, a nucleotide sequence coding for the entire signal sequence and a nucleotide sequence that specifies the three amino-terminal amino acids of extracellular α-amylase, and is bounded at the carboxy terminus coding region by a synthetic linker to which is fused the hsa nucleotide sequence.

5. A process as claimed in claim 3 or claim 4, wherein said synthetic linker comprises a HindIII synthetic linker.

6. A process as claimed in claim 3 or claim 4, wherein, prior to ligation with said segment of amy[BamP] gene, said hsa sequence is derived from a preproHSA structural gene, which is modified by means comprising

a) in vitro mutagenesis to create a restriction enzyme recognition site within the pro-coding sequence of said structural gene;

b) digestion with a restriction enzyme which recognizes said site; and

c) ligation to a synthetic oligonucleotide containing a restriction enzyme recognition site which can be cleaved by a restriction enzyme to provide said hsa gene with an amino terminus coding region which is complementary to a synthetic linker at the carboxy terminus coding region of the amy[BamP] gene segment.

7. A process as claimed in claim 6, wherein said preproHSA structural gene is modified by in vitro mutagenesis to create an XhoI site within the pro-coding sequence, followed by digestion with XhoI and ligation to a synthetic oligonucleotide linker containing a HindIII site; said carboxy terminus coding region of the amy[BamP] gene segment is bounded by a HindIII synthetic linker, and said amy[BamP] gene segment and the modified hsa nucleotide sequence are digested with HindIII prior to ligation.

8. A process as claimed in claim 7 which further comprises modification of the initially produced gene fusion by oligonucleotide-directed mutagenesis to delete nucleotides which specify amino acids encoded by said HindIII synthetic linker and a portion of the HSA pro sequence such that the signal sequence coding region of the amy[BamP] gene is fused directly to a nucleotide sequence encoding mature HSA.

9. A process as claimed in claim 7 which further comprises modification of the initially produced gene fusion by oligonucleotide-directed mutagenesis such that eight base pairs of the HindIII synthetic linker are deleted and fourteen base pairs are substituted therefore such that the signal sequence coding region of the amy[BamP] gene is ligated directly to a nucleotide sequence encoding proHSA.

10. A process as claimed in claim 1, wherein the expression elements and signal sequence coding region comprise a segment of a neutral protease gene (npr[BamP]) from Bacillus amyloliquefaciens and the Bacillus host is B. subtilis.

11. A process as claimed in claim 10, wherein the segment of npr[BamP] gene comprises a promoter, a ribosome binding site, a translation initiation codon, a nucleotide sequence coding for the entire signal sequence and a nucleotide sequence which specifies the amino-terminal two amino acids of the "pro" region of the protease, and is bounded at its carboxy terminus coding region by a synthetic linker to which is fused the hsa nucleotide sequence.

12. A process as claimed in claim 11, wherein prior to ligation with said segment of npr[BamP] gene, said hsa sequence is derived from a preproHSA structural gene, which is modified by means comprising

a) in vitro mutagenesis to create a restriction enzyme recognition site within the pro-coding sequence of said structural gene:

b) digestion with a restriction enzyme which recognizes said site; and

c) ligation to a synthetic oligonucleotide containing a restriction enzyme recognition site which can be cleaved by a restriction enzyme to provide said hsa gene with an amino terminus coding region which is complementary to a synthetic linker at the carboxy terminus coding region of the npr[BamP] gene segment.

13. A process as claimed in claim 12, wherein said preproHSA structural gene is modified by in vitro mutagenesis to create an XhoI site within the pro-coding sequence, followed by digestion with XhoI and ligation to a synthetic oligonucleotide linker containing a HindIII site; said carboxy terminus coding region of the npr[BamP] gene segment is bounded by a HindIII synthetic linker, and said npr[BamP] gene segment and the modified hsa nucleotide sequence are digested with HindIII prior to ligation.

14. A process as claimed in any one of claims 1 to 13, wherein said nucleotide sequence encoding HSA comprises DNA encoding a translation stop codon and wherein a transcription terminator is placed near the DNA specifying said translational termination codon, on the promoter-distal side.

15. A process for preparing a vector comprising a gene fusion as claimed in any one of claims 1 to 14, which comprises incorporating said gene fusion in a plasmid.

16. A process as claimed in claim 15, wherein said gene fusion is incorporated in a plasmid comprising a functional replicon for said Bacillus host.

17. A process as claimed in claim 16 wherein the vector prepared is selected from plasmids pGX2464, pGX1812, pGX2468, pGX3804, pGX3805, pGX3821, pGX3812 and pGX3837.

18. A strain of Bacillus transformed by a vector prepared by a process as claimed in any one of claims 15 to 17.

19. A transformed strain as claimed in claim 18 wherein said gene fusion is inserted in the chromosomal DNA.

20. A Bacillus strain having the identifying characteristics of GX7003.

21. A Bacillus strain having the identifying characteristics of GX4129.

22. A Bacillus strain having the identifying characteristics of GX7034.

23. A Bacillus strain having the identifying characteristics of GX7033.

24. A Bacillus strain having the identifying characteristics of GX7097.

25. A Bacillus strain having the identifying characteristics of GX7080 (deposited with the ATCC as 53443).

26. A method for producng a protein having the antigenic characteristics of HSA which comprises cultivating a transformed Bacillus strain as claimed in any one of claims 18 to 25 in a nutrient medium under conditions for the production of said protein.

27. A method as claimed in claim 26 wherein said protein is secreted into said nutrient medium.

28. A method as claimed in claim 27 wherein said nutrient medium comprises about 2% yeast extract.

29. A method as claimed in any one of claims 26 to 28 wherein a transformed Bacillus strain as claimed in any one of claims 18 to 25 is chemically mutagenized prior to being grown in a nutrient medium so as to result in a higher level of production of said protein.

30. A method as claimed in claim 29 wherein said transformed strain is mutagenized with nitrosoguanidine.

31. A strain having the identifying characteristics of GX5528 (deposited with the ATCC as 53444).

32. A strain having the identifying characteristics of GX5552 (deposited with the ATCC as 53442).

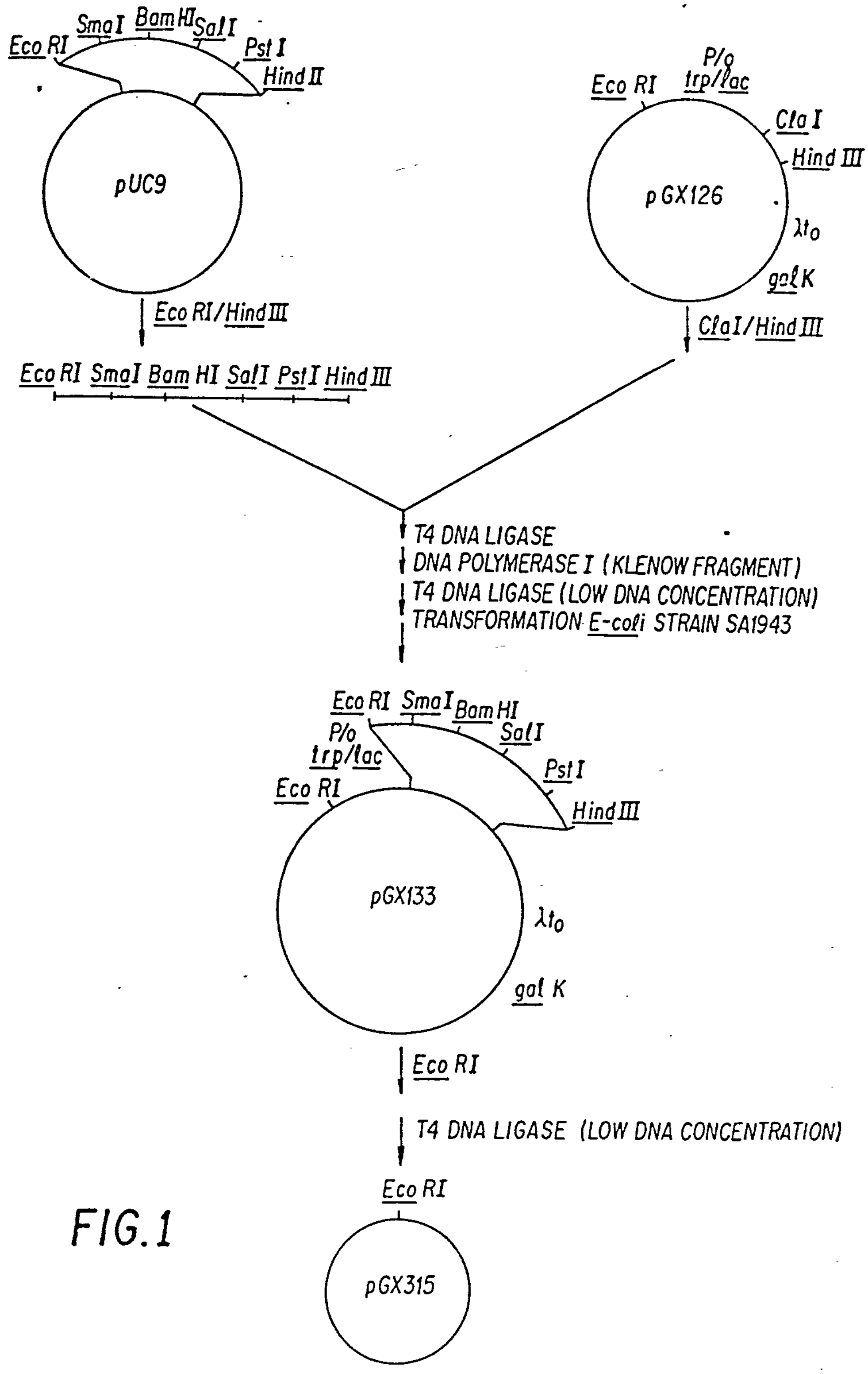
Eco RI
Sma I
Bam HI
Sal I
Pst I
Hind II
pUC9
Eco RI/Hind III
P/o
trp/lac
Eco RI
Cla I
Hind III
pGX126
λt0
gal K
Cla I/Hind III
Eco RI Sma I Bam HI Sal I Pst I Hind III
T4 DNA LIGASE
DNA POLYMERASE I (KLENOW FRAGMENT)
T4 DNA LIGASE (LOW DNA CONCENTRATION)
TRANSFORMATION E-coli STRAIN SA1943
Eco RI
Sma I
Bam HI
Sal I
P/o
trp/lac
Pst I
Eco RI
Hind III
pGX133
λt0
gal K
Eco RI
T4 DNA LIGASE (LOW DNA CONCENTRATION)
Eco RI
pGX315

FIG.1

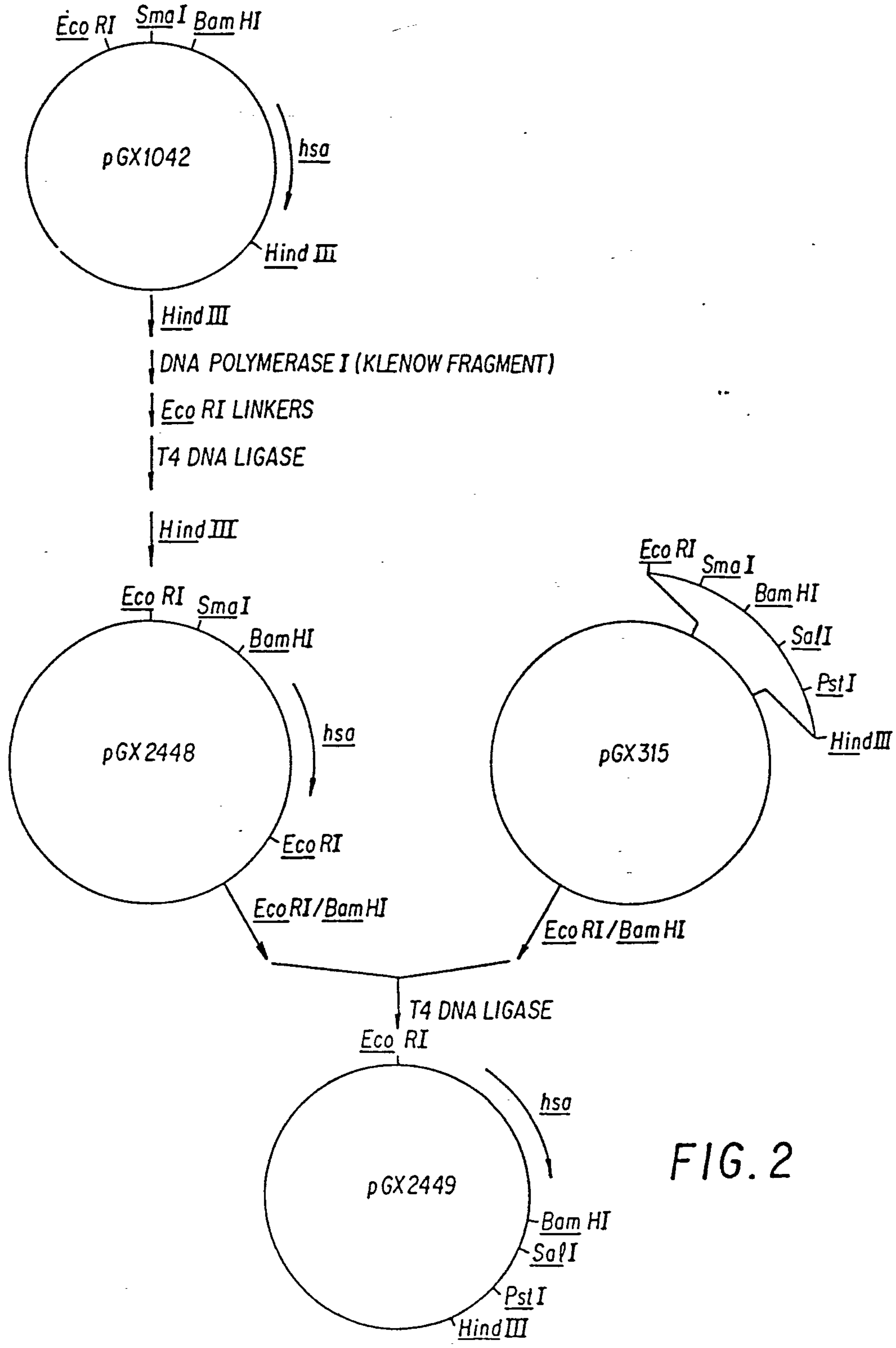
Eco RI
Sma I
Bam HI
pGX1042
hsa
Hind III
Hind III
DNA POLYMERASE I (KLENOW FRAGMENT)
Eco RI LINKERS
T4 DNA LIGASE
Hind III
Eco RI
Sma I
Bam HI
pGX2448
hsa
Eco RI
Eco RI
Sma I
Bam HI
Sal I
Pst I
Hind III
pGX315
Eco RI/Bam HI
Eco RI/Bam HI
T4 DNA LIGASE
Eco RI
hsa
pGX2449
Bam HI
Sal I
Pst I
Hind III

FIG. 2

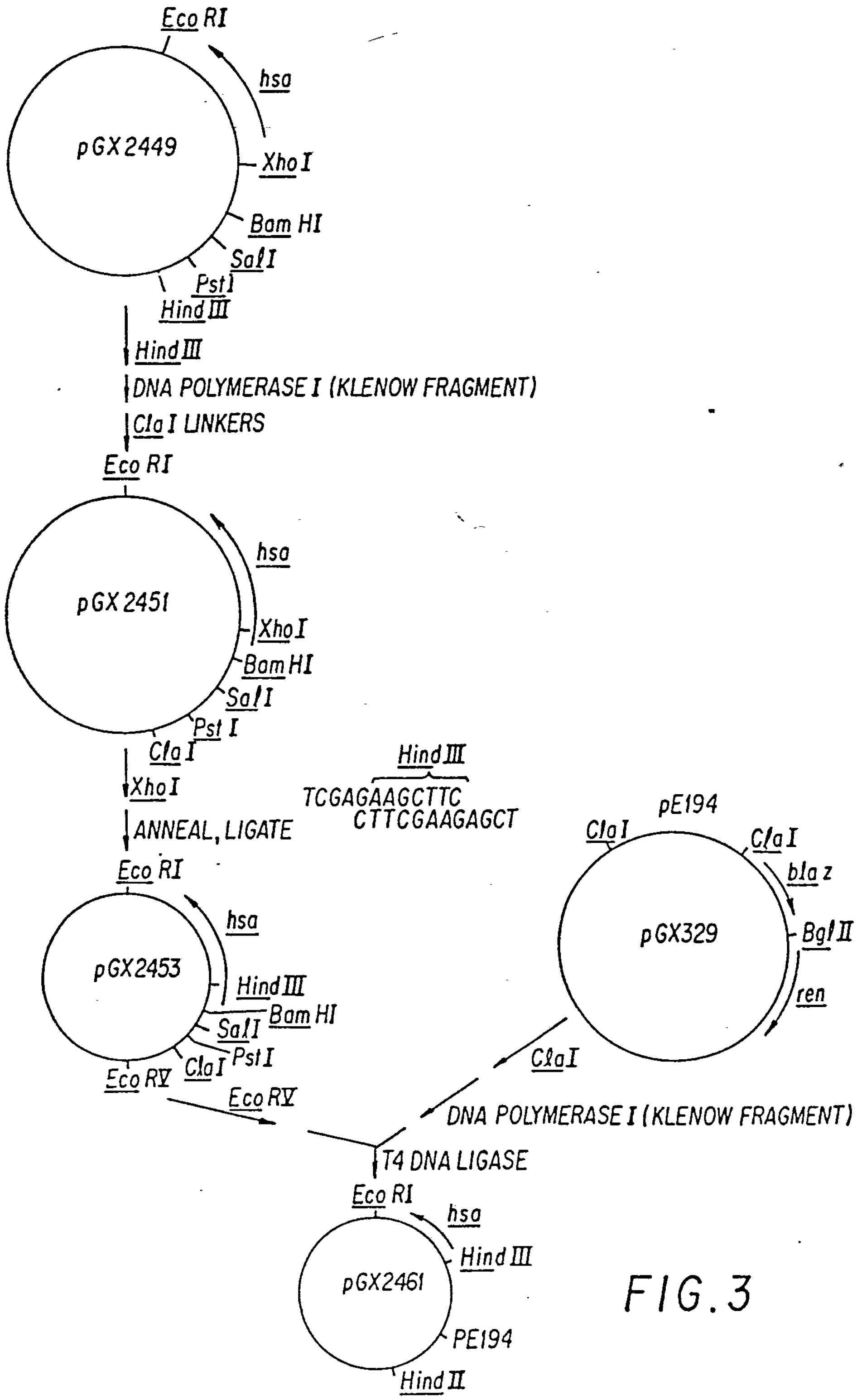
Eco RI
hsa
pGX 2449
Xho I
Bam HI
Sal I
Pst I
Hind III
Hind III
DNA POLYMERASE I (KLENOW FRAGMENT)
Cla I LINKERS
Eco RI
hsa
pGX 2451
Xho I
Bam HI
Sal I
Pst I
Cla I
Xho I
ANNEAL, LIGATE
Hind III
TCGAGAAGCTTC
CTTCGAAGAGCT
Eco RI
hsa
pGX2453
Hind III
Bam HI
Sal I
Pst I
Cla I
Eco RV
Eco RV
pE194
Cla I
Cla I
bla z
pGX329
Bgl II
ren
Cla I
DNA POLYMERASE I (KLENOW FRAGMENT)
T4 DNA LIGASE
Eco RI
hsa
Hind III
pGX2461
PE194
Hind II

FIG. 3

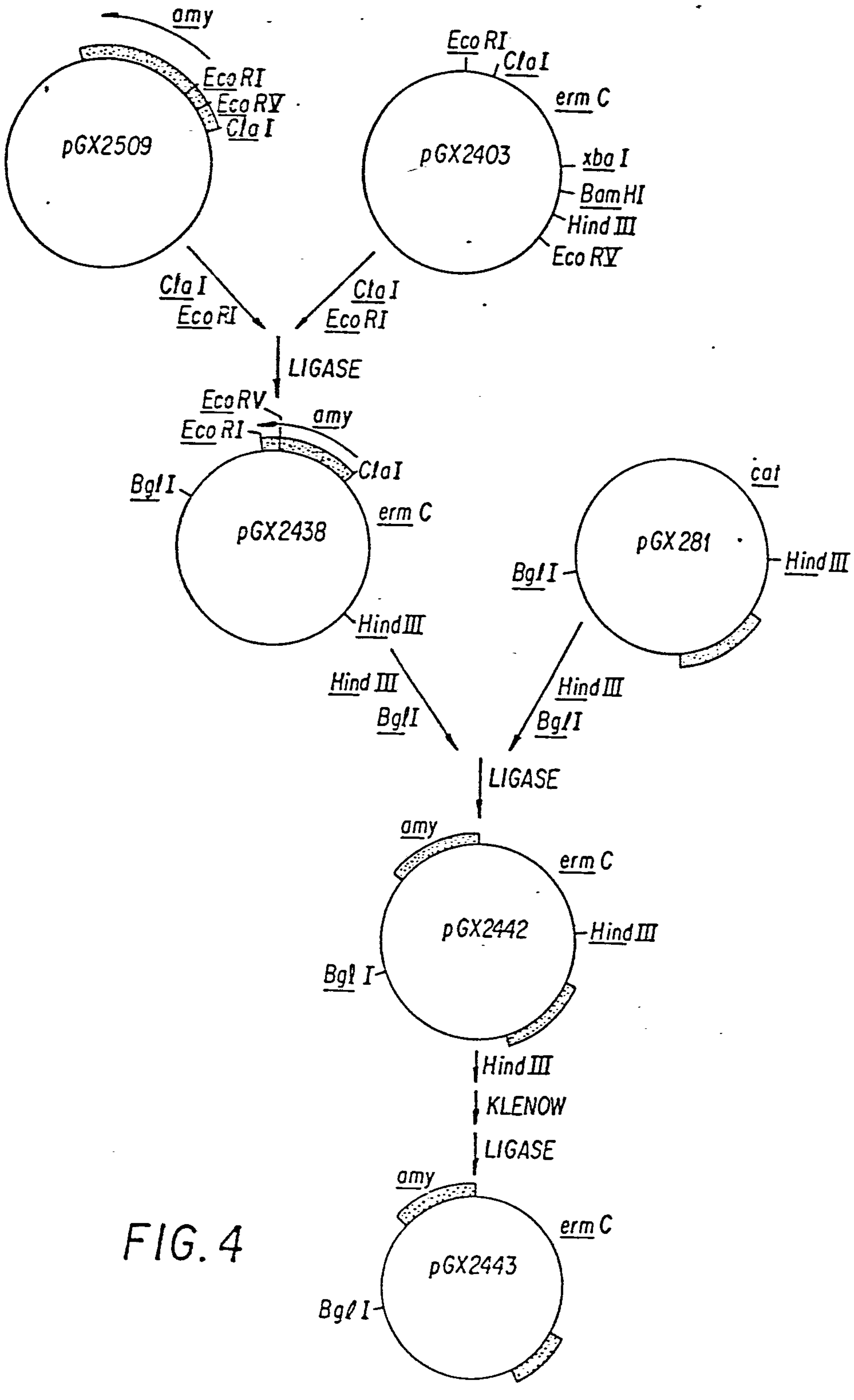

amy
EcoRI
EcoRV
ClaI
pGX2509
EcoRI
ClaI
ermC
pGX2403
xbaI
BamHI
HindIII
EcoRV
ClaI
EcoRI
ClaI
EcoRI
LIGASE
EcoRV
EcoRI
amy
ClaI
BglI
pGX2438
ermC
HindIII
cat
pGX281
BglI
HindIII
HindIII
BglI
HindIII
BglI
LIGASE
amy
ermC
pGX2442
HindIII
BglI
HindIII
KLENOW
LIGASE
amy
ermC
pGX2443
BglI


FIG. 4

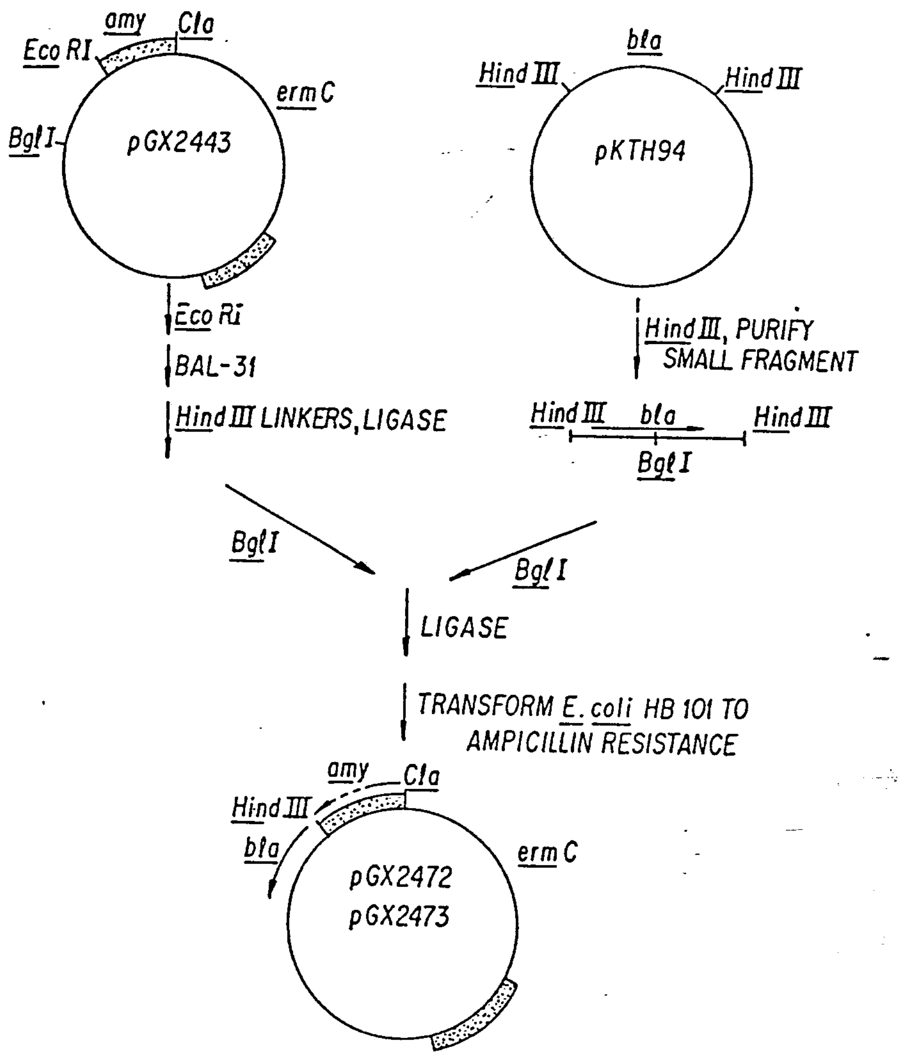
amy
Cla
Eco RI
erm C
Bgl I
pGX2443
bla
Hind III
Hind III
pKTH94
Eco RI
BAL-31
Hind III LINKERS, LIGASE
Hind III, PURIFY
SMALL FRAGMENT
Hind III
bla
Hind III
Bgl I
Bgl I
Bgl I
LIGASE
TRANSFORM E. coli HB 101 TO
AMPICILLIN RESISTANCE
amy
Cla
Hind III
bla
erm C
pGX2472
pGX2473

FIG. 5

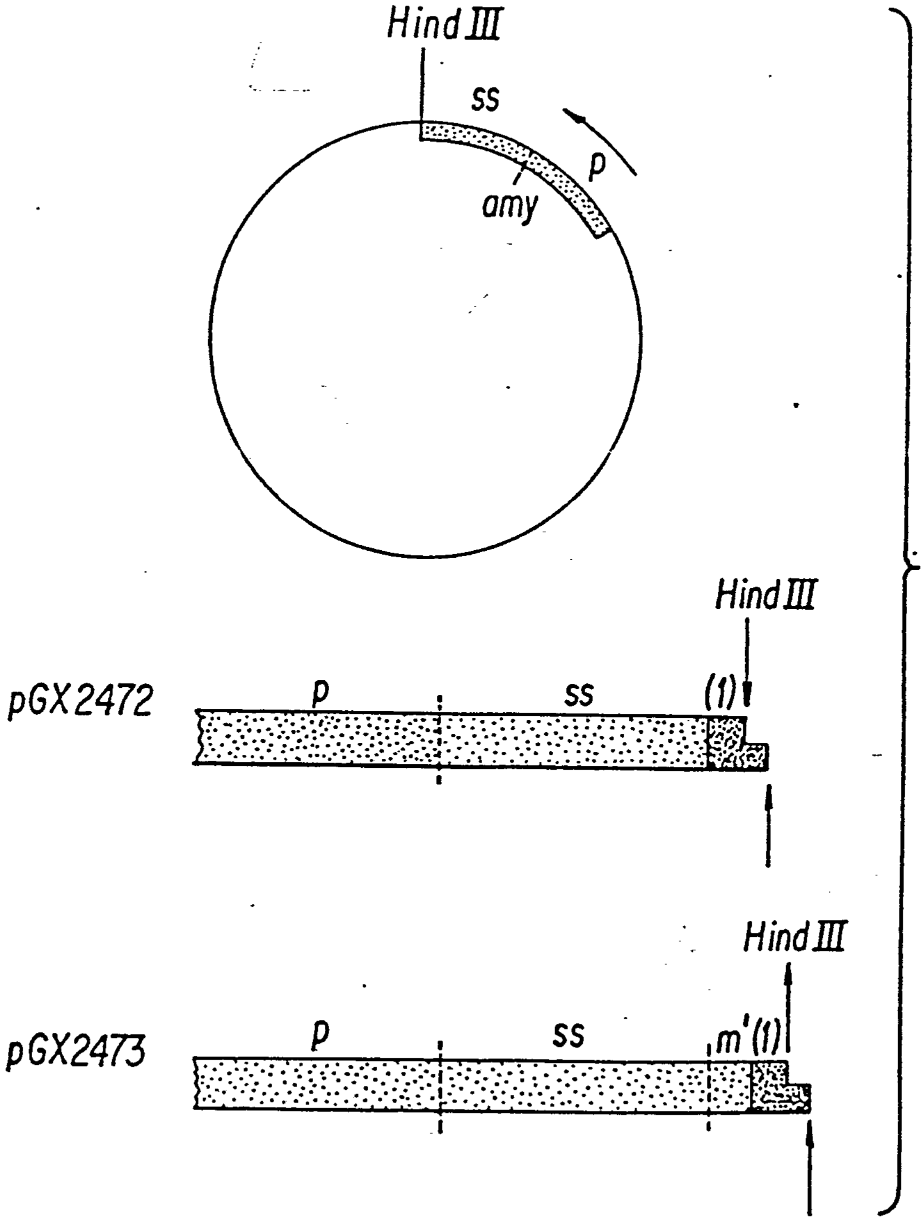
Hind III
ss
p
amy
Hind III
pGX2472
p
ss
(1)
Hind III
pGX2473
p
ss
m'(1)

FIG. 6

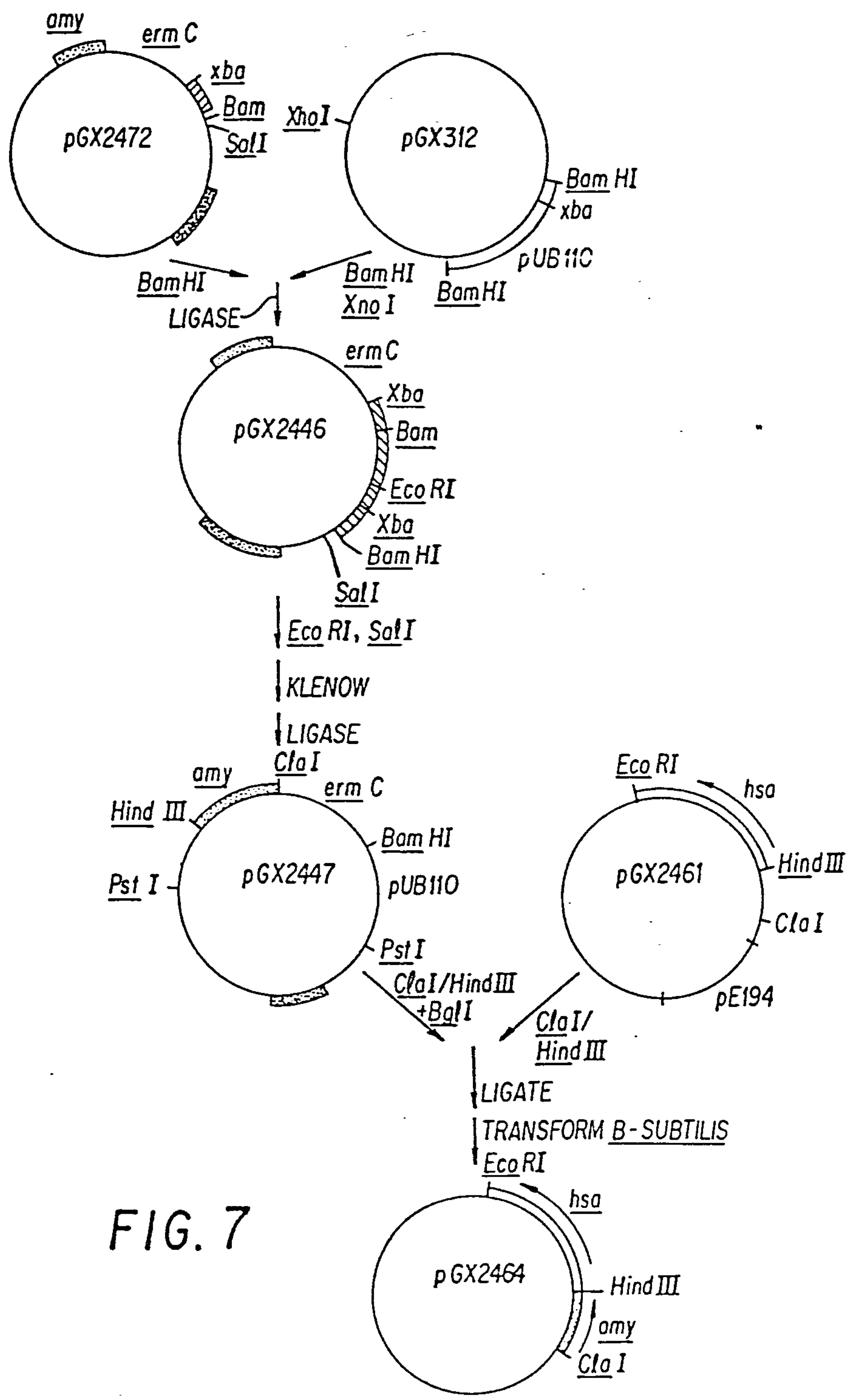
amy
erm C
xba
Bam
Sal I
pGX2472
Xho I
pGX312
Bam HI
xba
pUB110
Bam HI
Bam HI
LIGASE
Bam HI
Xno I
erm C
pGX2446
Xba
Bam
Eco RI
Xba
Bam HI
Sal I
Eco RI, Sal I
KLENOW
LIGASE
Cla I
amy
erm C
Hind III
Bam HI
Pst I
pGX2447
pUB110
Pst I
Cla I/Hind III
+Bgl I
Eco RI
hsa
Hind III
pGX2461
Cla I
pE194
Cla I/
Hind III
LIGATE
TRANSFORM B-SUBTILIS
Eco RI
hsa
pGX2464
Hind III
amy
Cla I

FIG. 7

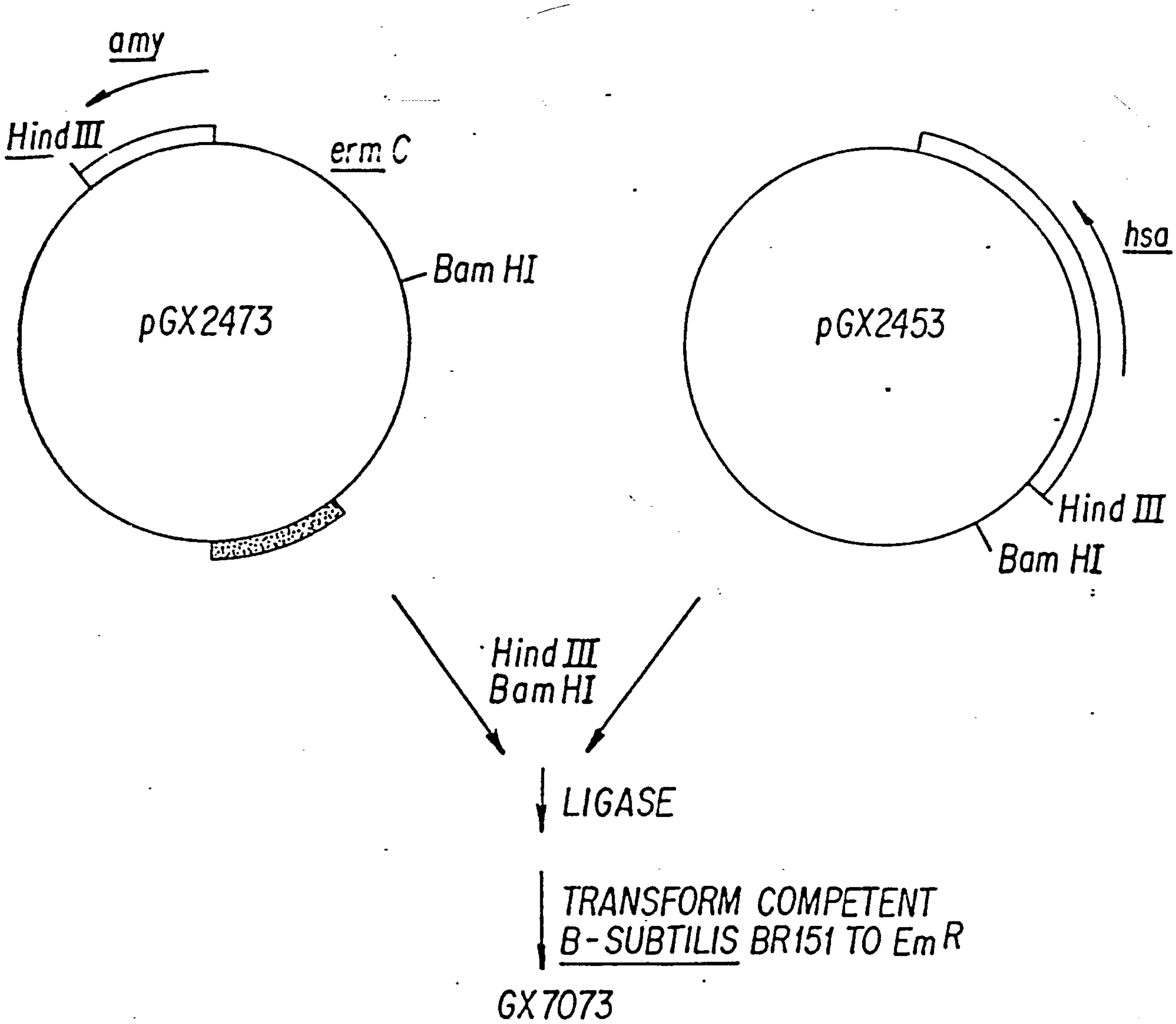
amy
Hind III
erm C
Bam HI
pGX2473
hsa
pGX2453
Hind III
Bam HI
Hind III
Bam HI
LIGASE
TRANSFORM COMPETENT
B-SUBTILIS BR151 TO Em R
GX7073

FIG. 8

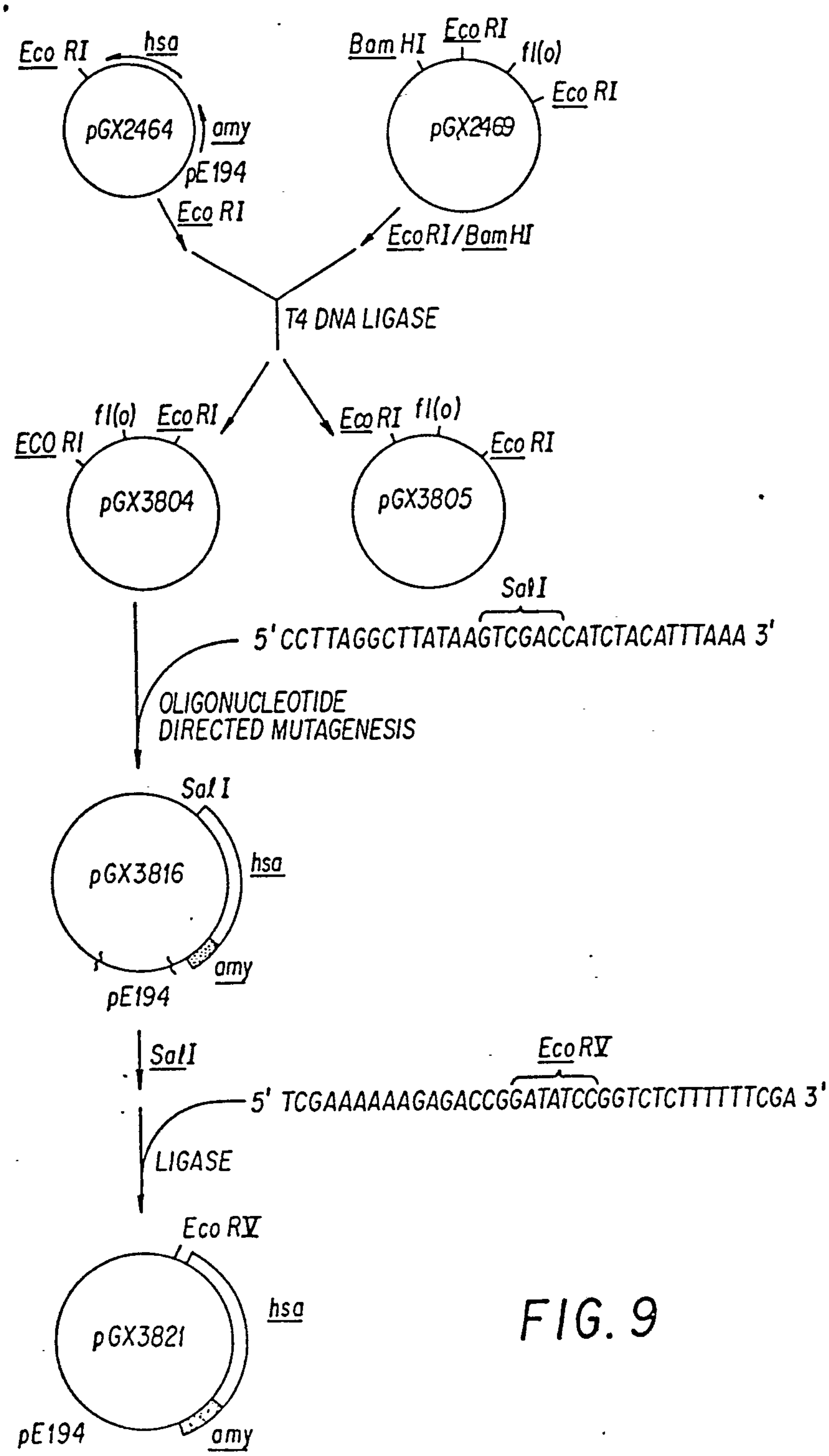
Eco RI
hsa
pGX2464
amy
pE194
Bam HI
Eco RI
f1(o)
Eco RI
pGX2469
Eco RI
EcoRI/BamHI
T4 DNA LIGASE
ECO RI
f1(o)
EcoRI
pGX3804
EcoRI
f1(o)
Eco RI
pGX3805
SalI
5' CCTTAGGCTTATAAGTCGACCATCTACATTTAAA 3'
OLIGONUCLEOTIDE
DIRECTED MUTAGENESIS
Sal I
pGX3816
hsa
amy
pE194
SalI
EcoRV
5' TCGAAAAAAGAGACCGGATATCCGGTCTCTTTTTTCGA 3'
LIGASE
Eco RV
pGX3821
hsa
pE194
amy

FIG. 9

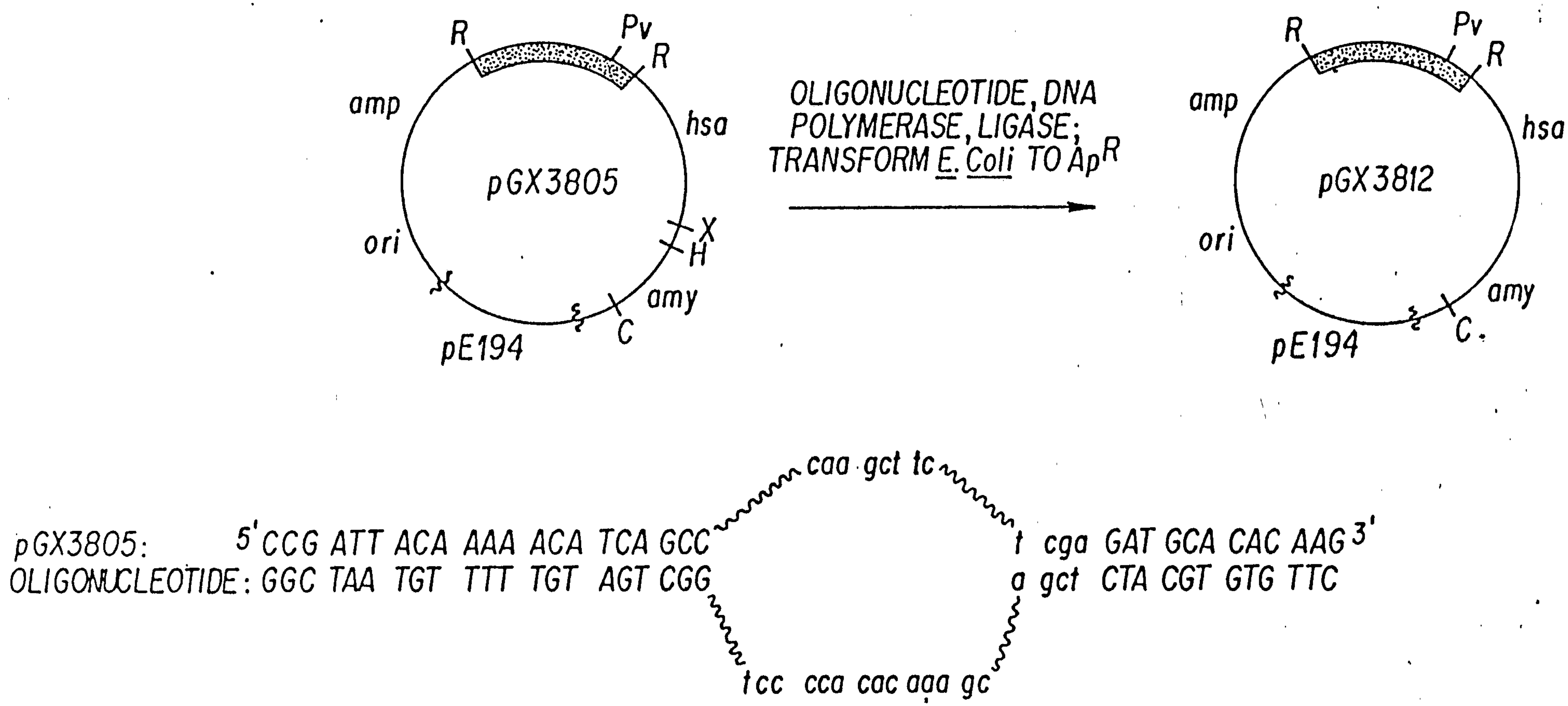
R
Pv
R
amp
hsa
pGX3805
ori
X
H
amy
pE194
C
OLIGONUCLEOTIDE, DNA
POLYMERASE, LIGASE;
TRANSFORM E. Coli TO ApR
R
Pv
R
amp
hsa
pGX3812
ori
amy
pE194
C.
caa gct tc
pGX3805: 5'CCG ATT ACA AAA ACA TCA GCC
t cga GAT GCA CAC AAG 3'
OLIGONUCLEOTIDE: GGC TAA TGT TTT TGT AGT CGG
a gct CTA CGT GTG TTC
tcc cca cac aaa gc

FIG. 10

DIAGRAM OF THE STRUCTURES OF FOUR
amy/hsa FUSIONS

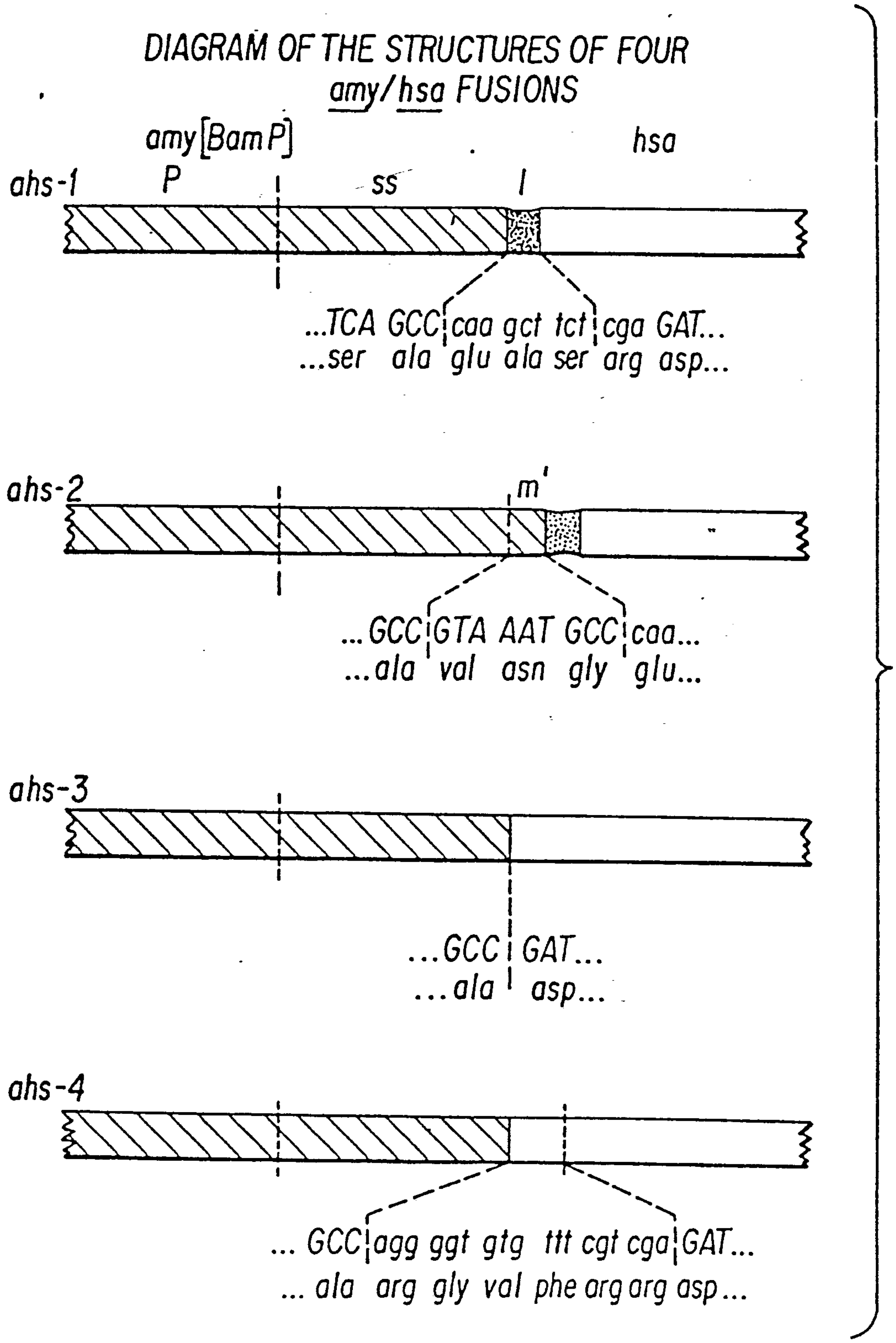


FIG. 11

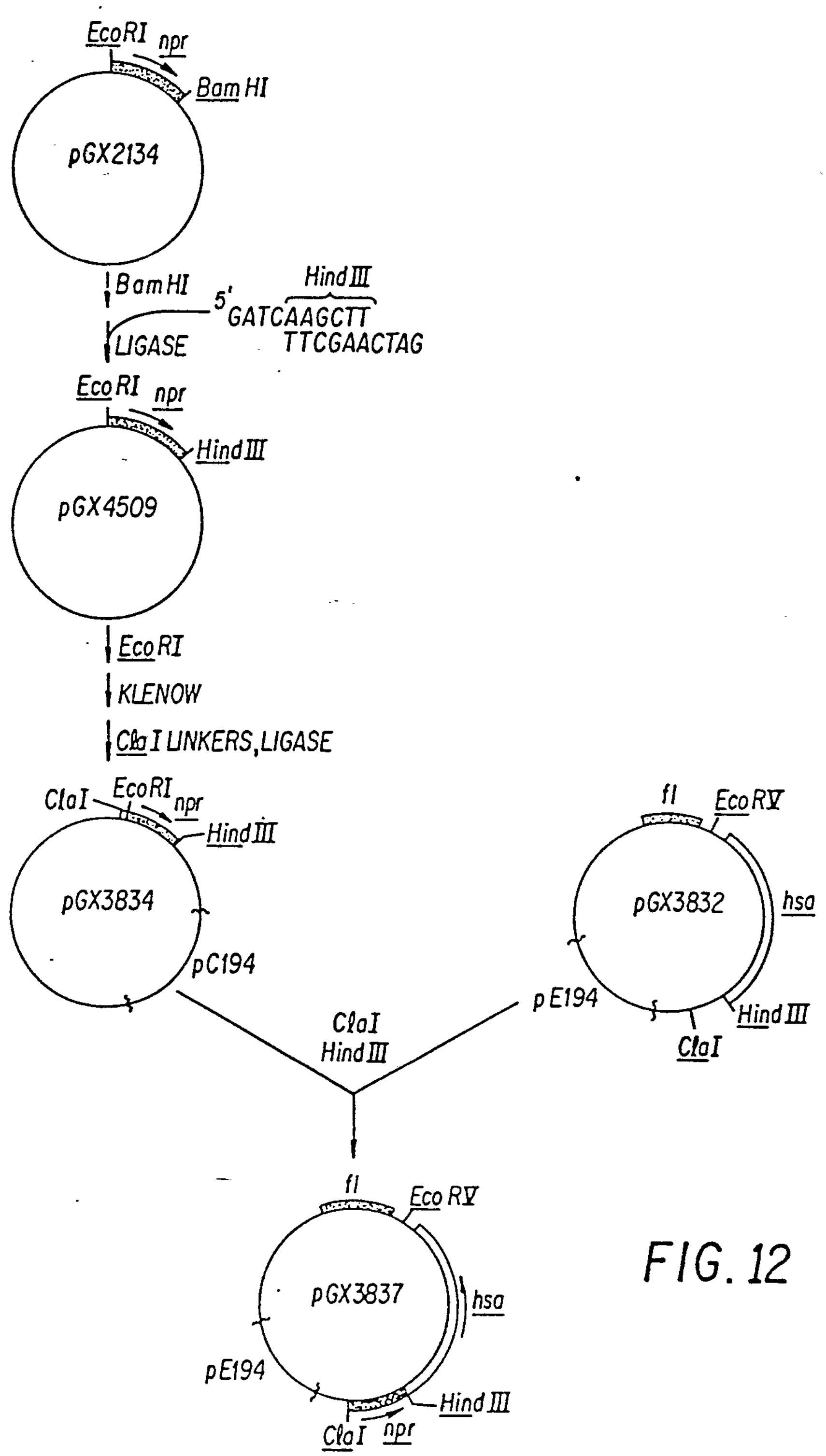
EcoRI
npr
BamHI
pGX2134
BamHI
5'
GATCAAGCTT
TTCGAACTAG
HindIII
LIGASE
EcoRI
npr
HindIII
pGX4509
EcoRI
KLENOW
ClaI LINKERS, LIGASE
ClaI
EcoRI
npr
HindIII
pGX3834
pC194
fI
EcoRV
hsa
pGX3832
pE194
HindIII
ClaI
ClaI
HindIII
fI
EcoRV
pGX3837
hsa
pE194
HindIII
ClaI
npr

FIG. 12

Neu eingereicht / Newly filed
Nouvellement déposé



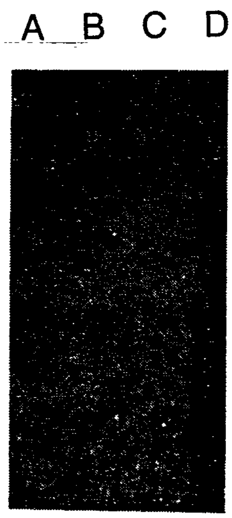


FIG. 13

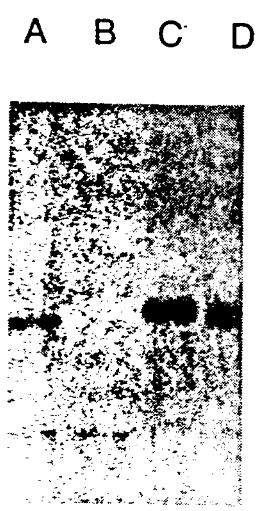


FIG. 14

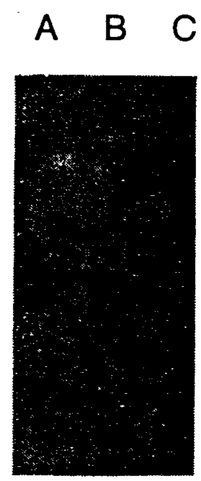


FIG. 15

Neu eingereicht / Newly filed
Nouvellement déposé



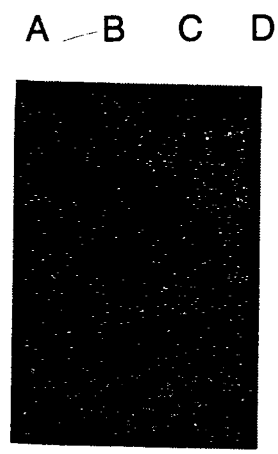


FIG. 16

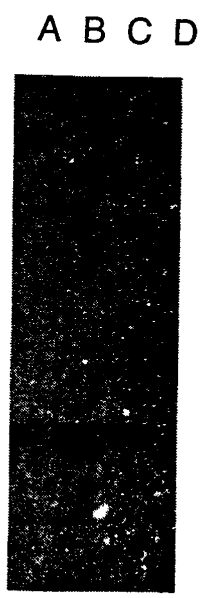


FIG. 17

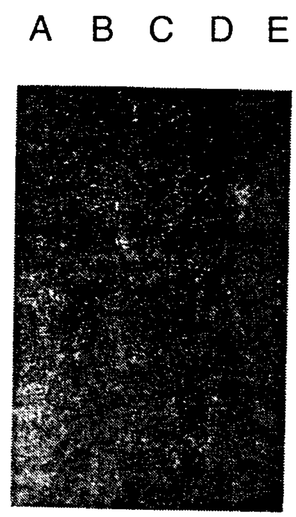


FIG. 18